(19) Europäisches Patentamt
European Patent Office
Office européen des brevets

(11) Publication number: 0 103 918 B1

(12) # EUROPEAN PATENT SPECIFICATION

(45) Date of publication of patent specification: 29.07.87

(51) Int. Cl.$^4$: C 07 C 145/04, C 07 D 213/64, A 01 N 47/34

(21) Application number: 83201173.8

(22) Date of filing: 09.08.83

(54) **Pesticidal benzoylurea compounds.**

(30) Priority: 16.09.82 GB 8226386
22.03.83 GB 8307919

(43) Date of publication of application:
28.03.84 Bulletin 84/13

(45) Publication of the grant of the patent:
29.07.87 Bulletin 87/31

(84) Designated Contracting States:
AT BE CH DE FR GB IT LI LU NL SE

(56) References cited:
GB-A-1 167 127
US-A-3 862 955

(73) Proprietor: SHELL INTERNATIONALE RESEARCH MAATSCHAPPIJ B.V.
Carel van Bylandtlaan 30
NL-2596 HR Den Haag (NL)

(72) Inventor: Anderson, Martin
92 Clare Road
Whitstable Kent C7 52GH (GB)

(74) Representative: Hunter, Keith Roger Ian et al
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 103 918 B1

**Description**

The present invention relates to benzoylurea compounds and their preparation, to compositions containing such compounds, and to their use as pesticides.

GB 1,460,410 and GB 1,501,607 disclose that certain disubstituted ureas have pesticidal activity.

The Applicants have now discovered that a further class of novel urea derivatives have pesticidal activity. These ureas are trisubstituted, and contain a specifically-substituted phenylthio group.

Accordingly, the present invention provides a benzoylurea compound of formula:—

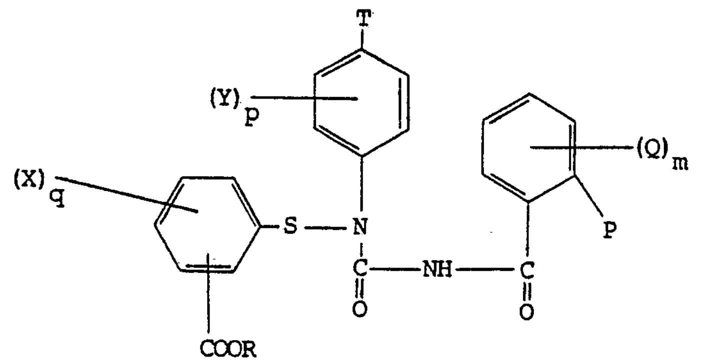 (I)

in which each of P and Q independently represents a halogen atom or an alkyl group; m represents 0, 1 or 2; R represents an optionally-substituted alkyl, alkenyl, alkynyl, cycloalkyl or phenyl group, a hydrogen atom, one equivalent of an alkali metal or alkaline earth metal, an ammonium or substituted ammonium group, or a group of general formula $—(CH_2CH_2O)_nR^1$ in which $R^1$ represents an optionally substituted alkyl, alkenyl, alkynyl, cycloalkyl or phenyl group, and n represents 1, 2, 3, 4 or 5; each X independently represents a halogen atom, a cyano, nitro or carboxy group, or an alkyl, alkoxy, alkylthio, cycloalkyl, cycloalkyloxy, cycloalkylthio, alkenyl, alkenylthio, alkylcarbonyl, alkoxycarbonyl, alkylsulphonyl, alkynyl, phenyl, phenoxy, phenylthio or amino group; q = 0, 1, 2, 3, or 4; T represents a halogen atom, an alkyl, haloalkyl, alkoxy, haloalkoxy, alkenyloxy or haloalkenyloxy group, or a phenoxy or pyridyloxy group optionally substituted by one or more substituents selected from halogen atoms and nitro, alkyl, haloalkyl and cyano groups; each Y independently represents a halogen atom or a nitro, cyano, alkyl or haloalkyl group; and p = 0, 1, 2, 3 or 4; the optional substituents in an optionally substituted alkyl, alkenyl or alkynyl moiety being selected from halogen atoms; alkoxy, haloalkoxy, hydroxy, carboxy, alkoxycarbonyl and cycloalkyl groups; saturated or unsaturated heterocyclic groups containing one oxygen, sulphur or nitrogen atom, and having 5 or 6 atoms in the ring; groups of formula $—O—(CH_2CH_2O)_n$alkyl where n is 1 to 4; and optionally substituted phenyl groups; the optional substituents in an optionally substituted cycloalkyl moiety being selected from alkyl and haloalkyl groups and those substituent groups listed in respect of alkyl; and the optional substituents in an optionally substituted phenyl group being selected from halogen atoms and alkyl, haloalkyl, alkoxy, haloalkoxy, alkenyl, haloalkenyl, nitro and cyano groups; an alkyl, alkenyl or alkynyl moiety having up to 6 carbon atoms; a cycloalkyl group having 3 to 7 ring carbon atoms; except that when R represents an optionally substituted alkyl group the alkyl moiety may contain 1 to 20 carbon atoms, and when $R^1$ represents an alkyl or haloalkyl group, the halogen being chlorine or fluorine, the alkyl or haloalkyl group may contain 1 to 10 carbon atoms.

Except where otherwise stated, throughout this Specification and claims, alkyl, alkenyl and alkynyl moieties have up to 6, especially up to 4, carbon atoms, and cycloalkyl groups having 3 to 7 ring carbon atoms. Halogen atoms may be iodine, bromine, chlorine or fluorine atoms, with fluorine and chlorine atoms being preferred. A heterocyclyl group contains one oxygen, sulphur or nitrogen atom, may be saturated or unsaturated, and has 5 or 6 atoms in the ring.

Optional substituents which may be present in an optionally substituted alkyl, alkenyl or alkynyl moiety are selected from halogen atoms, alkoxy, haloalkoxy, hydroxy, carboxy, alkoxycarbonyl, cycloalkyl and heterocyclyl groups, groups of formula $—O—(CH_2CH_2O)_n$alkyl where n is 1 to 4, and optionally substituted phenyl groups. These substituents may also be present in optionally substituted cycloalkyl groups, along with alkyl and haloalkyl groups. Optional substituents which may be present in optionally substituted phenyl, phenoxy and pyridyloxy groups are selected from halogen atoms and alkyl, haloalkyl, alkoxy, haloalkoxy, alkenyl, haloalkenyl, nitro and cyano groups.

Preferably each of P and Q independently represents a C(1—4) alkyl, especially methyl, group, or a fluorine, chlorine or bromine atom, and m is 0 or 1. If m is 1, Q is preferably in the 6 position of the phenyl ring.

Preferably T represents a bromine or, especially, fluorine or chlorine atom; a C(1—4) alkyl, haloalkyl or haloalkoxy group, especially a methyl, trifluoromethyl or trifluoromethoxy group; an optionally substituted phenoxy group in which the optional substituents are selected from C(1—4) alkyl and haloalkyl, especially methyl and trifluoromethyl, groups, fluorine and chlorine atoms, and nitro and cyano groups, or an

optionally substituted pyridyloxy group in which the optional substituents are selected from trifluoromethyl groups and fluorine and chlorine atoms.

When T contains a pyridyloxy group, the pyridyl moiety is preferably a 2-pyridyl moiety; if this 2-pyridyl moiety is substituted, it preferably bears halogen or haloalkyl, especially trifluoromethyl, substituents, preferably in the 3-, 5- or 3,5- positions.

When T is a trifluoromethylphenoxy group this is preferably a 4-trifluoromethylphenoxy group and, when the phenoxy moiety thereof is further substituted, this substitution is preferably in the 2-position. Thus 2-chloro-4-trifluoromethyphenoxy, 2-fluoro-4-trifluoromethylphenoxy and 2-cyano-4-trifluoromethylphenoxy are preferred examples of the group T.

Preferably p = 1 when T is a 4-substituted phenoxy group, or p = 0 when T is a 4-substituted-phenoxy group bearing a second substituent.

Preferably Y represents a fluorine, chlorine or bromine atom, a C(1—4) alkyl or haloalkyl group, or a nitro or cyano group. Preferably p is 0, 1 or 2.

Preferably X represents a bromine or, especially, chlorine or fluorine atom, or a C(1—4) alkyl group. Preferably q is 0 or 1, especially 0.

Preferably R represents an optionally substituted alkyl group containing 1 to 20, especially 1 to 15, carbon atoms, a C(3—6) cycloalkyl group, or an optionally substituted phenyl group. Preferred substituents in an optionally substituted phenyl group R are selected from C(1—4) alkyl and haloalkyl groups, such as methyl and trifluoromethyl groups, nitro and cyano groups, and halogen atoms, especially fluorine and chlorine atoms.

Preferred substituents in an optionally substituted alkyl group R are halogen atoms, especially chlorine and fluorine atoms; C(1—4) alkoxy groups, especially methoxy and ethoxy groups; carboxy groups; alkoxycarbonyl groups containing 2 to 5 carbon atoms, for example ethoxycarbonyl and methoxycarbonyl groups; groups of formula $-O-(CH_2CH_2O)_n$alkyl where n is 1 to 4, especially 1, and the alkyl moiety has 1 to 4, especially 1 or 2, carbon atoms; optionally substituted phenyl groups in which the preferred optional substituents are as given above for an optionally substituted phenyl group R; and heterocyclic groups, especially tetrahydrofuryl groups.

In one preferred embodiment of the invention, R represents an unsubstituted alkyl group having 1 to 20 carbon atoms, especially 1 to 10 carbon atoms, most preferably 1 to 5 carbon atoms.

In a further preferred embodiment of the invention, R represents a group of general formula

$$-(CH_2CH_2O)_nR^1 \qquad (II)$$

in which $R^1$ represents an optionally-substituted alkyl, alkenyl, alkynyl, cycloalkyl or phenyl group, and n represents 1, 2, 3, 4 or 5. Preferably $R^1$ represents an alkyl or haloalkyl group containing 1 to 10 carbon atoms, the halogen being chlorine or fluorine. Most preferably, $R^1$ represents an alkyl group having 1 to 4 carbon atoms, for example methyl, ethyl, isopropyl or tertiary butyl. Preferably n is 1 or 2. Preferably the group $-COO(CH_2CH_2O)_nR^1$ is located on the 2-position of the phenyl ring of the phenylthio group.

Thus typical benzoylureas according to the invention have the general formula I in which each of P and Q independently represents a fluorine, chlorine or bromine atom or an alkyl group containing 1 to 4 carbon atoms and m represents 0 or 1; R represents an alkyl, haloalkyl or alkoxyalkyl group containing 1 to 15, especially 1 to 10, carbon atoms and in which the halogen substituent is chlorine or fluorine and the alkoxy substituent contains 1 to 4 carbon atoms; one equivalent of an alkali metal or alkaline earth metal; or an ammonium or a C(1—6)alkyl-substituted-ammonium group; each X represents a fluorine, chlorine or bromine atom, or an alkyl group of 1 to 4 carbon atoms and q = 0, 1, 2, 3 or 4; T represents a fluorine, chlorine or bromine atom, a haloalkyl or haloalkoxy group of 1 to 4 carbon atoms in which the halogen substituent is fluorine, chlorine and/or bromine, or a trihalomethyl-, nitro-, or cyano-phenoxy group in which the phenoxy moiety can also bear one or more fluorine, chlorine or bromine, C(1—4)alkyl, nitro or cyano substituents; each Y independently represents a fluorine, chlorine, or bromine atom, a nitro or cyano group, or a C(1—4)alkyl or haloalkyl group in which the halogen substituent is fluorine, chlorine or bromine; and p = 0, 1, 2, 3 or 4.

More preferred benzoylureas according to the invention have the general formula I in which each of P and Q independently represents a fluorine, chlorine or bromine atom; m represents 0 or 1; R represents a C(1—10) alkyl or haloalkyl group in which the halogen substituent is fluorine or chlorine; q = 0; T represents a chlorine atom, a fluoroalkyl or fluoroalkoxy group of 1 to 4 carbon atoms and 1 to 5 fluorine atoms, or a trifluoromethylphenoxy group in which the phenoxy moiety can also bear one or more fluorine, chlorine or bromine atoms; each Y independently represents a fluorine or chlorine atom; and p = 0, 1, 2, 3 or 4.

The —COOR substituent in the phenylthio group of the benzoylureas of formula I may be located in any one of the available positions on the phenyl ring but it is preferably located in the 2-position.

The invention also provides a process for preparing the novel benzoylurea compounds, which comprises reacting a benzoylisocyanate of formula:

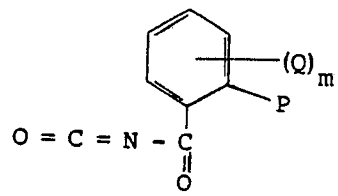

(III)

with a sulphenamide of formula:

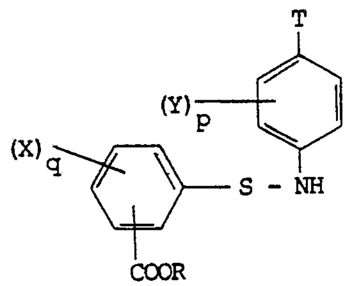

(IV)

in which P, Q, m, R, X, q, T, Y and p have the meanings specified above.

The reaction is suitably carried out in the presence of a solvent. Suitable solvents are aromatic solvents such as benzene, toluene, xylene, or chlorobenzene, hydrocarbons such as petroleum fractions, chlorinated hydrocarbons such as chloroform, methylene chloride or dichloroethane, and ethers such as diethylether, dibutylether, or dioxan. Mixtures of solvents are also suitable.

Preferably the reaction is carried out at a temperature from 0°C to 100°C, suitably ambient temperature. Preferably the molar ratio of benzoyl-isocyanate to sulphenamide is from 1:1 to 2:1. Preferably the reaction is carried out under anhydrous conditions.

The sulphenamide intermediates IV are themselves novel and constitute a further aspect of the invention; they may be prepared by reacting a sulphenyl halide of formula:

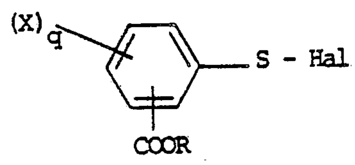

(V)

wherein X, q and R have the meanings specified above in relation to formula I and Hal represents a halogen atom, preferably a chlorine or bromine atom, with an aniline of formula:

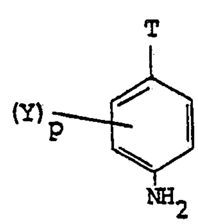

(VI)

wherein T, Y and p have the meanings specified above in relation to formula I in the presence of a dehydrohalogenating agent, such as an organic or inorganic base. Suitable bases include alkali metal hydroxides, amides or alkoxides, for example, sodium ethoxide; alkali metal or alkaline earth metal hydroxides, for example, potassium hydroxide; and primary, secondary or tertiary amines, for example, triethylamine or piperidine.

The reaction is conveniently carried out in the presence of an aprotic solvent; suitable solvents include aromatic solvents such as benzene, toluene, xylene or chlorobenzene, hydrocarbons such as petroleum, chlorinated hydrocarbons, such as methylene chloride, and ethers such as diethylether and dibutylether.

The reaction is suitably carried out at a temperature in the range of −10° to 50°C, preferably −5 to 20°C.

The sulphenyl halide of formula V may be prepared from the corresponding thio or disulphide, e.g. according to the method of L. Katz, L. S. Karger, W. Schroeder and M. S. Cohen, J. Org. Chem. 1953, *18*, 1380. In the preparation of the intermediates IV the sulphenyl halide V starting material may be prepared *in situ*. The disulphide starting material for the sulphenyl halide of formula V may be prepared by the esterification of the corresponding dicarboxylic acid according to the method of J. C. Grivas, J. Org. Chem. 1975, *40*, 2029.

The compounds of the present invention have been found to have high insecticidal and acaricidal activity. Accordingly the present invention also provides pesticidal compositions comprising a compound of formula I as defined above together with a carrier. Such a composition may contain a single compound or a mixture of several compoounds of the invention. The invention further provides a method of combating pests at a locus, which comprises applying an insecticidally effective amount of a benzoylurea compound or composition according to the present invention to the locus.

Unlike the compounds of the prior art, which are virtually insoluble in organic solvents and therefore extremely difficult to formulate without great expense, the compounds of the general formula I are in general readily soluble in organic solvents making formulation relatively simple, and the resulting compositions easy to handle and economically attractive. The compounds of the general formula I also appear to possess a faster mode of action than the compounds of the prior art.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contains 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricuultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Grandules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is

substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or the oil-in-water type, amd may have a thick 'mayonnaise'-like consistency.

The composition of the invention may also contain other ingredients, for example, other compounds possessing pesticidal properties. Further insecticidal compounds may be included, particularly such compounds having a different mode of activity.

The following Examples illustrate the invention. Example A is concerned with the synthesis of a novel sulphenamide starting material, Examples 1—63 are concerned with the synthesis of the novel benzoylureas, and Example 64 with the biological activity of the novel benzoylureas. Examples 65 to 114 give details of novel intermediates of the general formula IV.

Example A

Preparation of isopropyl 2-[[[4-(trifluoromethoxy)phenyl]amino]thio]benzoate

A solution of bromine (0.65ml) in dry methylene chloride (10ml) was added over thirty minutes to a stirred solution of diisopropyl 2,2'-dithiobisbenzoate (4.9g) in the same solvent (40ml) at ambient temperature. The resulting dark red solution was stirred at this temperature for 1.25 hours and was then added over thirty minutes to a stirred solution of 4-(trifluoromethoxy)aniline (4.45g) in dry methylene chloride (100ml) containing triethylamine (5.0ml). The temperature of the reaction mixture was kept at 5—10°C throughout the addition by means of an ice bath and when the addition had been completed, the reaction was allowed to warm to ambient temperature over 4.0 hours. The solvent was removed under reduced pressure and the residue was suspended in diethyl ether (250ml) and washed with water to remove triethylamine hydrobromide. The resulting diethyl ether solution was dried using magnesium sulphate and evaporated to yield the crude product which was purified by crystallisation from diethyl ether/light petroleum.

The crystallisation yielded 7.4g of the desired product as colourless plates, melting point 120—121°C. The following analytical results were obtained:—

| | | | |
|---|---|---|---|
| Calculated: | C: 55.0% | H: 4.3% | N: 3.8% |
| Found: | C: 55.0% | H: 4.5% | N: 3.8% |

Example 1

Preparation of isopropyl 2-[[[[(2-chlorobenzoyl)amino]carbonyl][4-trifluoromethoxy)phenyl]amino]thio]benzoate

A solution of 2-chlorobenzoyl isocyanate (2.7g) in dry toluene (5.0ml) was added to a stirred suspension of isopropyl 2-[[[4-(trifluoromethoxy)phenyl]amino]thio]benzoate (3.7g) in the same solvent (20.0ml) at room temperature. After 18 hours, a cake of crystals had formed. The reaction mixture was cooled to −5°C and filtered, washing the crystals with cold toluene followed by light petroleum. After drying at 40°C under vacuum, the pure product was obtained as colourless crystals (4.8g). Melting point 143—144°C.

The following analytical results were obtained:—

| | | | |
|---|---|---|---|
| Calculated: | C: 54.3% | H: 3.7% | N: 5.1% |
| Found: | C: 54.5 | H: 3.5% | N: 5.2% |

Example 2

Preparation of butyl 2-[[[[(2,6-difluorobenzoyl)amino]carbonyl][4-(trifluoromethyl)phenyl]amino]thio]benzoate

A solution of 2,6-difluorobenzoyl isocyanate (1.1g) in dry toluene (5.0ml) was added to a stirred solution of butyl 2-[[[4-(trifluoromethyl)phenyl]amino]thio]benzoate (1.9g) in the same solvent (7.0ml) at room temperature. After 18 hours, the reaction mixture was cooled to −5°, and the crystalline precipitate was separated, washing with cold toluene followed by light petroleum. The pure product was obtained as colourless crystals (2.5g melting point 79—81°) after recrysallisation from diethyl ether/light petroleum.

The following analytical results were obtained:—

| | | | |
|---|---|---|---|
| Calculated: | C: 56.5% | H: 3.8% | N: 5.1% |
| Found: | C: 56.3% | H: 4.0% | N: 5.1% |

Example 3

Preparation of propyl 2[[[4-[2-chloro-4-(trifluoromethyl)-phenoxyphenyl][[(2,6-difluorobenzoyl)amino]carbonyl]aminothio]benzoate

A solution of 2,6-difluorobenzoyl isocyanate (1.8g) in dry toluene (5.0ml) was added to a stirred solution of propyl 2-[[[[2-chloro-4-(trifluoromethyl)phenoxy]phenyl]amino]thio]benzoate (3.6g) in the same

solvent (7.0ml) at room temperature. After 4.0 hours, the reaction mixture was evaporated under reduced pressure and the product was isolated from the residue by rapid chromatography on silica gel using methylene chloride as eluent. Crystallisation from diethyl ether/light petroleum afforded the pure product as colourless crystals (4.2g) melting point 148—150°.

The following analytical results were obtained:—

| | | | |
|---|---|---|---|
| Calculated: | C: 56.0% | H: 3.3% | N: 4.2: |
| Found: | C: 56.3% | H: 3.3% | N:4.3% |

## Example 4

Preparation of ethyl 2-[[[[(2-chlorobenzoyl)amino]carbonyl][4-[2-chloro-4-(trifluoromethyl)phenoxy]phenyl]amino]thio]benzoate

A solution of 2-chlorobenzoyl isocyanate (1.8g) in dry toluene (5.0ml) was added to a stirred solution of ethyl 2-[[[[2-chloro-4-(trifluoromethyl)phenoxy]phenyl]amino]thio]benzoate (3.5g) in the same solvent (7.0ml) at room temperature. After 18.0 hours, the reaction mixture was diluted with dry light petroleum (30ml) and the resulting precipitate of crude product was separated, washing with light petroleum. This material was purified by rapid chromatography on silica gel followed by crystallisation from diethyl ether/light petroleum.

The crystallisation yielded 3.1g of the desired product as colourless crystals, melting point 98—100°C.

The following analytical results were obtained:—

| | | | |
|---|---|---|---|
| Calculated: | C: 55.5% | H: 3.2% | N: 4.3% |
| Found: | C: 55.6% | H: 3.2% | N: 4.3% |

## Examples 5 to 63

In Examples 5 to 63 compounds were prepared by one of the methods described in Examples 1 to 4. The compounds, their melting points and chemical analyses are shown in Table 1 below.

TABLE 1

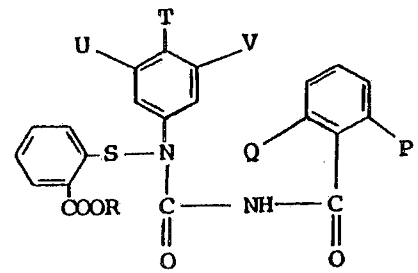

| Example No. | T | R | P | Q | U | V | m.p. (°C) | | Analysis % C | H | N |
|---|---|---|---|---|---|---|---|---|---|---|---|
| 5 | 2-Cl-4-$CF_3$—$C_6H_3$—O— | Sec-$C_4H_9$— | F | F | H | H | 77—79 | Calc.<br>Found. | 56.6<br>56.8 | 3.5<br>3.9 | 4.1<br>4.0 |
| 6 | —$OCF_3$ | $CH_3$ | CL | H | H | H | 144—145 | Calc.<br>Found. | 52.6<br>52.7 | 3.1<br>2.9 | 5.3<br>4.8 |
| 7 | —$OCF_3$ | $C_2H_5$ | CL | H | H | H | 150—152 | Calc.<br>Found. | 53.5<br>53.6 | 3.4<br>3.2 | 5.2<br>5.3 |
| 8 | —$OCF_3$ | n-$C_3H_7$ | CL | H | H | H | 122—123 | Calc.<br>Found. | 54.3<br>54.5 | 3.7<br>3.6 | 5.1<br>5.2 |
| 9 | —$OCF_3$ | n-$C_4H_9$ | CL | H | H | H | 111—113 | Calc.<br>Found. | 55.1<br>55.1 | 3.9<br>3.7 | 4.9<br>5.0 |

TABLE 1 (continued)

| Example No. | T | R | P | Q | U | V | m.p. (°C) | | Analysis % C | H | N |
|---|---|---|---|---|---|---|---|---|---|---|---|
| 10 | $—OCF_3$ | $—CH(CH_3)CH_2CH_3$ | Cl | H | H | H | 118—119 | Calc.<br>Found. | 55.1<br>54.8 | 3.9<br>3.7 | 4.9<br>5.0 |
| 11 | $—OCF_3$ | n-$C_5H_{11}$ | Cl | H | H | H | 88—89 | Calc.<br>Found | 55.8<br>55.9 | 4.2<br>3.7 | 4.8<br>5.2 |
| 12 | $—OCF_3$ | $—CH(CH_3)CH_2CH_2CH_3$ | Cl | H | H | H | 119—120 | Calc.<br>Found. | 55.8<br>55.8 | 4.2<br>3.8 | 4.8<br>5.0 |
| 13 | $—CF_3$ | $CH_3$ | F | F | H | H | 161—163 | Calc.<br>Found. | 54.1<br>54.8 | 2.9<br>3.5 | 5.5<br>5.4 |
| 14 | $—CF_3$ | $C_2H_5$ | F | F | H | H | 137—140 | Calc.<br>Found. | 55.0<br>55.4 | 3.2<br>3.1 | 5.3<br>5.5 |
| 15 | $—CF_3$ | n-$C_3H_7$ | F | F | H | H | 140—143 | Calc.<br>Found. | 55.8<br>55.5 | 3.5<br>3.4 | 5.2<br>5.3 |
| 16 | $—CF_3$ | i-$C_3H_7$ | F | F | H | H | 155—157 | Calc.<br>Found. | 55.8<br>55.7 | 3.5<br>3.4 | 5.2<br>5.4 |
| 17 | $—CF_3$ | $(CH_2)_2CH(CH_3)_2$ | F | F | H | H | 139—141 | Calc.<br>Found. | 57.2<br>58.1 | 4.1<br>4.0 | 5.0<br>4.9 |

TABLE 1 (continued)

| Example No. | T | R | P | Q | U | V | m.p. (°C) | | Analysis % C | H | N |
|---|---|---|---|---|---|---|---|---|---|---|---|
| 18 | $—CF_3$ | $CH_2CF_3$ | F | F | H | H | 80—82 | Calc.<br>Found. | 49.8<br>50.4 | 2.4<br>2.4 | 4.8<br>4.8 |
| 19 | $2\text{-Cl-4-}CF_3—C_6H_3—O—$ | $CH_3$ | F | F | H | H | 135—137 | Calc.<br>Found. | 54.7<br>54.9 | 2.8<br>2.8 | 4.4<br>4.5 |
| 20 | $2\text{-Cl-4-}CF_3—C_6H_3—O—$ | $C_2H_5$ | F | F | H | H | 126—128 | Calc.<br>Found. | 55.3<br>55.5 | 3.1<br>3.0 | 4.3<br>4.3 |
| 21 | $2\text{-Cl-4-}CF_3—C_6H_3—O—$ | $n\text{-}C_4H_9$ | F | F | H | H | 124—126 | Calc.<br>Found. | 56.6<br>56.7 | 3.5<br>3.4 | 4.1<br>4.2 |
| 22 | $2\text{-Cl-4-}CF_3—C_6H_3—O—$ | $—CH(CH_3)CH_2CH_3$ | F | F | H | H | 77—79 | Calc.<br>Found. | 56.6<br>56.8 | 3.5<br>3.9 | 4.1<br>4.0 |
| 23 | $2\text{-Cl-4-}CF_3—C_6H_3—O—$ | $n\text{-}C_5H_{11}$ | F | F | H | H | 63—65 | Calc.<br>Found. | 57.2<br>56.7 | 3.8<br>3.5 | 4.0<br>4.1 |
| 24 | $2\text{-Cl-4-}CF_3—C_6H_3—O—$ | $—CH(CH_3)CH_2CH_2CH_3$ | F | F | H | H | 70—72 | Calc.<br>Found. | 57.2<br>57.2 | 3.8<br>3.9 | 4.0<br>4.3 |
| 25 | $2\text{-Cl-4-}CF_3—C_6H_3—O—$ | $CH_3$ | Cl | H | H | H | 139—141 | Calc.<br>Found. | 54.8<br>54.3 | 3.0<br>3.1 | 4.4<br>4.6 |

TABLE 1 (continued)

| Example No. | T | R | P | Q | U | V | m.p. (°C) | | Analysis % C | H | N |
|---|---|---|---|---|---|---|---|---|---|---|---|
| 26 | 2-Cl-4-$CF_3$—$C_6H_3$—O— | n-$C_3H_7$ | Cl | H | H | H | 135—137 | Calc.<br>Found. | 56.1<br>56.1 | 3.5<br>3.6 | 4.2<br>4.2 |
| 27 | 2-Cl-4-$CF_3$—$C_6H_3$—O— | n-$C_4H_9$ | Cl | H | H | H | 119—121 | Calc.<br>Found. | 56.7<br>56.7 | 3.7<br>3.4 | 4.1<br>4.1 |
| 28 | 2-Cl-4-$CF_3$—$C_6H_3$—O— | —$(CH_2)_2CH(CH_3)_2$ | F | F | H | H | Gum | Calc.<br>Found. | 57.2<br>56.7 | 3.8<br>4.1 | 4.0<br>3.8 |
| 29 | 2-Cl-4-$CF_3$—$C_6H_3$—O— | —$(CH_2)_9CH_3$ | F | F | H | H | 48—52 | Calc.<br>Found. | 59.8<br>59.5 | 4.7<br>5.0 | 3.7<br>3.6 |
| 30 | 2-Cl-4-$CF_3$—$C_6H_3$—O— | —$(CH_2)_{13}CH_3$ | F | F | H | H | Gum | Calc.<br>Found. | 61.6<br>61.6 | 5.4<br>5.7 | 3.4<br>3.4 |
| 31 | 2-Cl-4-$CF_3$—$C_6H_3$—O— | —$(CH_2)_{15}CH_3$ | F | F | H | H | Gum | Calc.<br>Found. | 62.4<br>62.0 | 5.7<br>5.8 | 3.3<br>3.4 |
| 32 | 2-Cl-4-$CF_3$—$C_6H_3$—O— | cyclohexyl | F | F | H | H | 98—100 | Calc.<br>Found. | 57.4<br>58.3 | 3.7<br>4.1 | 4.0<br>3.9 |
| 33 | 2-Cl-4-$CF_3$—$C_6H_3$—O— | benzyl | F | F | H | H | 88—91 | Calc.<br>Found. | 59.0<br>58.8 | 3.1<br>3.0 | 3.9<br>3.8 |

TABLE 1 (continued)

| Example No. | T | R | P | Q | U | V | m.p. (°C) | | Analysis % C | H | N |
|---|---|---|---|---|---|---|---|---|---|---|---|
| 34 | 2-Cl-4-$CF_3$—$C_6H_2$—O— | phenyl | F | F | H | H | 96—99 | Calc.<br>Found. | 58.4<br>58.6 | 2.9<br>3.0 | 4.0<br>4.0 |
| 35 | 2-Cl-4-$CF_3$—$C_6H_3$—O— | —$CH(CH_3)CO_2C_2H_5$ | F | F | H | H | 81—84 | Calc.<br>Found. | 54.8<br>55.0 | 3.3<br>3.3 | 3.9<br>3.8 |
| 36 | 2-Cl-4-$CF_3$—$C_6H_3$—O— | —$CH_2CO_2C_2H_5$ | F | F | H | H | 78—80 | Calc.<br>Found. | 54.2<br>54.4 | 3.1<br>3.0 | 4.0<br>3.9 |
| 37 | 2-Cl-4-$CF_3$—$C_6H_3$—O— | 2-tetrahydrofuryl-methyl | F | F | H | H | 83—86 | Calc.<br>Found. | 56.1<br>55.8 | 3.4<br>3.4 | 4.0<br>4.2 |
| 38 | Cl | n-$C_3H_7$ | F | F | H | H | 105—108 | Calc.<br>Found. | 57.1<br>56.6 | 3.8<br>3.6 | 5.6<br>5.6 |
| 39 | Cl | Sec-$C_4H_9$ | F | F | H | H | 75—78 | Calc.<br>Found. | 57.9<br>57.7 | 4.1<br>4.1 | 5.4<br>5.4 |
| 40 | $OCF_3$ | n-$C_3H_7$ | F | Cl | H | H | 124—125 | Calc.<br>Found. | 52.6<br>52.7 | 3.3<br>3.2 | 4.9<br>4.0 |
| 41 | $OCF_3$ | Sec-$C_4H_9$ | F | Cl | H | H | 99—100 | Calc.<br>Found. | 53.4<br>53.7 | 3.6<br>3.9 | 4.8<br>4.7 |

TABLE 1 (continued)

| Example No. | T | R | P | Q | U | V | m.p. (°C) | | Analysis % C | H | N |
|---|---|---|---|---|---|---|---|---|---|---|---|
| 42 | $OCF_3$ | n-$C_3H_7$ | F | F | H | H | 98—100 | Calc.<br>Found. | 54.1<br>53.7 | 3.4<br>3.5 | 5.0<br>5.3 |
| 43 | $OCF_3$ | Sec-$C_4H_9$ | F | F | H | H | 109—110 | Calc.<br>Found. | 54.9<br>55.1 | 3.7<br>4.0 | 4.9<br>4.9 |
| 44 | $CF_3$ | $C_2H_5$ | F | Cl | H | H | 145—147 | Calc.<br>Found. | 53.3<br>53.4 | 3.2<br>3.3 | 5.2<br>5.2 |
| 45 | 2-Cl-4-$CF_3$—$C_6H_3$—O— | —$CH_2CH_2OCH_3$ | F | F | H | H | 85—86 | Calc.<br>Found. | 54.7<br>54.9 | 3.2<br>3.1 | 4.1<br>4.2 |
| 46 | 2-Cl-4-$CF_3$—$C_6H_3$—O— | —$CH_2CH_2OC_2H_5$ | F | F | H | H | 64—66 | Calc.<br>Found. | 55.3<br>55.2 | 3.5<br>3.2 | 4.0<br>4.3 |
| 47 | 2-Cl-4-$CF_3$—$C_6H_3$—O— | —$(CH_2CH_2O)_2CH_3$ | F | F | H | H | 86—87 | Calc.<br>Found. | 54.7<br>54.7 | 3.6<br>3.5 | 3.9<br>3.9 |
| 48 | 2-Cl-4-$CF_3$—$C_6H_3$—O— | —$(CH_2CH_2O)_2C_2H_5$ | F | F | H | H | 52—56 | Calc.<br>Found. | 55.2<br>54.9 | 3.8<br>3.9 | 3.8<br>3.9 |
| 49 | 3-Cl-4-CN—$C_6H_3$—O— | n—$C_3H_7$ | Cl | H | Cl | Cl | 111—114 | Calc.<br>Found. | 54.0<br>53.3 | 3.1<br>3.1 | 6.1<br>5.8 |

TABLE 1 (continued)

| Example No. | T | R | P | Q | U | V | m.p. (°C) | | Analysis % C | H | N |
|---|---|---|---|---|---|---|---|---|---|---|---|
| 50 | 3-Cl-4-CN—$C_6H_3$—O— | Sec-$C_4H_9$ | Cl | H | Cl | Cl | 99—101 | Calc.<br>Found. | 54.6<br>53.9 | 3.3<br>3.3 | 6.0<br>5.7 |
| 51 | 4-CN-$C_6H_4$—O— | n-$C_3H_7$ | Cl | H | Cl | H | 98—99 | Calc.<br>Found. | 60.0<br>59.6 | 3.7<br>3.6 | 6.8<br>6.6 |
| 52 | 4-CN-$C_6H_4$—O— | Sec-$C_4H_7$ | Cl | H | Cl | H | 114—116 | Calc.<br>Found. | 60.6<br>60.3 | 3.4<br>3.8 | 6.6<br>6.4 |
| 53 | 4-$NO_2$—$C_6H_4$—O— | n-$C_3H_7$ | Cl | H | Cl | Cl | 122—124 | Calc.<br>Found. | 53.4<br>53.4 | 3.3<br>3.2 | 6.2<br>6.1 |
| 54 | 3,5-dichloro-2-pyridyloxy | Sec-$C_4H_9$ | F | F | Cl | H | 112—115 | Calc.<br>Found. | 52.9<br>52.8 | 3.2<br>3.3 | 6.2<br>5.9 |
| 55 | 3,5-dichloro-2-pyridyloxy | n-$C_3H_7$ | F | F | Cl | H | 110—112 | Calc.<br>Found. | 52.2<br>52.5 | 3.0<br>3.1 | 6.3<br>5.9 |
| 56 | 3,5-dicholoro-2-pyridyloxy | Sec-$C_3H_9$ | F | F | Cl | Cl | 140—142 | Calc.<br>Found. | 50.3<br>49.9 | 2.9<br>3.0 | 59<br>6.0 |
| 57 | 3,5-dichloro-2-pyridyloxy | n-$C_3H_7$ | F | F | Cl | Cl | 158—160 | Calc.<br>Found. | 49.6<br>49.8 | 2.7<br>2.6 | 6.0<br>6.0 |

TABLE 1 (continued)

| Example No. | T | R | P | Q | U | V | m.p. (°C) | | Analysis % C | H | N |
|---|---|---|---|---|---|---|---|---|---|---|---|
| 58 | 4-$NO_2$—$C_6H_4$—O— | n-$C_3H_7$ | F | F | Cl | Cl | 115—118 | Calc.<br>Found. | 53.3<br>52.8 | 3.1<br>2.9 | 6.2<br>6.2 |
| 59 | 4-$NO_2$—$C_6$—$H_4$—O— | Sec-$C_4H_9$ | F | F | Cl | Cl | 113—116 | Calc.<br>Found. | 53.9<br>53.8 | 3.3<br>3.3 | 6.1<br>5.9 |
| 60 | 4-$NO_2$—$C_6$—$H_4$—O— | n-$C_5H_{11}$ | F | F | Cl | Cl | 105—108 | Calc.<br>Found. | 54.5<br>54.5 | 3.6<br>3.6 | 6.0<br>5.9 |
| 61 | 4-$NO_2$—$C_6H_4$—O— | n-$C_4H_9$ | F | F | Cl | Cl | 100—103 | Calc.<br>Found. | 53.9<br>54.2 | 3.3<br>3.4 | 6.1<br>5.9 |
| 62 | 4-$NO_2$—$C_6H_4$—O— | n-$C_4H_9$ | F | Cl | Cl | Cl | 147—149 | Calc.<br>Found. | 52.7<br>52.5 | 3.3<br>2.9 | 5.9<br>5.9 |
| 63 | 4-$NO_2$—$C_6H_4$—O— | n-$C_5H_{11}$ | F | Cl | Cl | Cl | 165 | Calc.<br>Found. | 53.3<br>53.6 | 3.5<br>3.4 | 5.8<br>5.8 |

Example 64

The insecticidal activity of the benzoylurea compounds of the invention was determined in the following tests.

*Test 1*

The insecticidal and ovicidal activities of the compounds of the invention were assessed employing the pests *Spodoptera littoralis* (S.l.), *Aedes aegypti* (A.a) and eggs of *Spodoptera littoralis* (S.l.ov).

The test methods used for each species appear below. In each case the tests were conducted under normal conditions (23°C ± 2°C; fluctuating light and humidity).

In each test an $LC_{50}$ for the compound was calculated from the mortality figures and compared with the corresponding $LC_{50}$ for ethyl parathion in the same tests. The results are expressed as toxicity indices thus:

$$\text{toxicity indices} = \frac{LC_{50}\ (\text{parathion})}{LC_{50}\ (\text{test compound})} \times 100$$

and are set out in Table 2 below.

(i) *Spodoptera Littoralis*

Solutions or suspensions of the compound were made up over a range of concentrations in 10% acetone/water containing .025% Triton X100 ("Triton" is a registered trade mark). These solutions were sprayed using a logarithmic spraying machine onto petri dishes containing a nutritious diet on which the *Spodoptera littoralis* larvae had been reared. When the spray deposit had dried each dish was infested with 10 2nd instar larvae. Mortality assessments were made 7 days after spraying.

(ii) *Aedes aegypti*

Several solutions of the test compound of varying concentration were prepared in acetone. 100 microlitre quantities were added to 100ml of tap water, the acetone being allowed to evaporate off. 10 early 4th instar larvae were placed in the test solution; after 48 hours the (surviving) larvae were fed with animal feed pellets, and the final percentage mortality assessed when all the larvae had either pupated and emerged as adults or died.

(iii) *Spodoptera littoralis* (ovicide)

Solutions as described in (i) above were prepared. Eggs less than 24 hours old were obtained as follows. Adult *Spodoptera littoralis* were held in large plastic cylinders containing blotting paper on which the moths laid their batches of eggs. Egg batches containing approximately 60—70 eggs were cut from the blotting paper with a 1cm surround. These were placed eggs uppermost on filter paper in the deeper half of 5cm disposable petri dishes and each batch of eggs was then sprayed with a different test solution or the control solution. The dishes were covered until the control eggs had hatched, approximately 5 days. The percentage ovicidal mortality was then calculated. In Table 2, a dash indicates no results available.

TABLE 2

| | Test Code | | |
|---|---|---|---|
| Compound of Example | S.l. | A.a. | S.l.ov |
| 1 | 490 | 160 | 830 |
| 2 | 160 | 72 | 3,100 |
| 3 | 2,400 | 160 | — |
| 4 | 2,450 | 130 | — |
| 5 | 2,400 | 250 | — |
| 6 | 190 | 210 | 670 |
| 7 | 74 | 130 | 390 |
| 8 | 150 | 72 | 970 |
| 9 | 400 | 150 | 840 |
| 10 | 88 | 72 | 400 |

TABLE 2 (continued)

| Compound of Example | Test Code S.I. | A.a. | S.I.ov |
|---|---|---|---|
| 11 | 106 | 76 | 900 |
| 12 | 131 | 64 | 630 |
| 13 | 120 | 100 | 625 |
| 14 | 150 | 220 | 1,200 |
| 15 | 180 | 140 | 1,100 |
| 16 | 75 | 150 | 2,700 |
| 17 | 188 | 140 | 620 |
| 18 | 81 | 62 | 100 |
| 19 | 1,900 | 400 | — |
| 20 | 2,200 | 240 | — |
| 21 | 1,800 | 300 | — |
| 22 | 2,400 | 250 | — |
| 23 | 2,320 | 160 | — |
| 24 | 1,690 | 4 | — |
| 25 | 1,500 | 170 | — |
| 26 | 1,400 | 95 | — |
| 27 | 1,900 | 71 | — |
| 28 | 2,300 | 650 | — |
| 29 | 3,700 | 230 | — |
| 30 | 940 | 110 | — |
| 31 | 1,700 | 12 | — |
| 32 | 1,500 | 1,500 | — |
| 33 | 1,700 | 16 | — |
| 34 | 2,200 | 1,000 | — |
| 35 | 1,900 | 460 | — |
| 36 | 2,300 | 300 | — |
| 37 | 1,100 | — | — |
| 38 | 49 | 110 | 2,400 |
| 39 | 49 | 130 | 500 |

TABLE 2 (continued)

| Compound of Example | Test Code S.I. | A.a. | S.I.ov |
|---|---|---|---|
| 40 | 270 | 76 | 39 |
| 41 | 210 | 79 | — |
| 42 | 160 | 130 | 2,500 |
| 43 | 150 | 250 | 860 |
| 44 | 94 | 82 | — |
| 45 | 2,500 | 780 | — |
| 46 | 1,450 | 850 | — |
| 47 | 1,860 | 860 | — |
| 48 | 1,180 | 670 | — |
| 49 | 430 | 110 | — |
| 50 | 120 | 110 | — |
| 51 | 170 | 85 | — |
| 52 | 75 | 88 | — |
| 53 | 1,200 | 150 | — |
| 54 | 960 | 1,500 | — |
| 55 | 1,000 | 240 | — |
| 56 | 2,100 | 1,400 | — |
| 57 | 1,100 | 350 | — |
| 58 | 2,000 | 580 | — |
| 59 | 1,200 | 530 | — |
| 60 | 1,800 | 500 | — |
| 61 | 2,100 | 210 | — |
| 62 | | | |
| 63 | | | |

Examples 65 to 114

Novel sulphenamide starting materials of the general formula IV were used in the synthesis of the compounds of Examples 1 to 63. Table 3 gives the physical data for each of these novel sulphenamides, which were prepared by the general method illustrated by Example A above.

TABLE 3

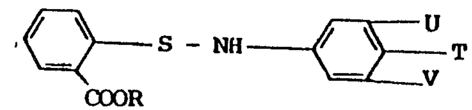

| Example No. | T | R | U | V | m.p. (°C) | | Analysis % C | H | N |
|---|---|---|---|---|---|---|---|---|---|
| 65 | $OCF_3$ | i-$C_3H_7$ | H | H | 120—121 | Calc. | 55.0 | 4.3 | 3.8 |
| | | | | | | Found. | 55.0 | 4.5 | 3.8 |
| 66 | $CF_3$ | n-$C_4H_9$ | H | H | 115—117 | Calc. | 58.5 | 4.9 | 3.8 |
| | | | | | | Found. | 58.4 | 4.9 | 3.8 |
| 67 | 2-Cl-4-$CF_3$—phenoxy | n-$C_3H_7$ | H | H | 117—119 | Calc. | 57.3 | 4.0 | 2.9 |
| | | | | | | Found. | 57.4 | 3.9 | 2.9 |
| 68 | 2-Cl-4-$CF_3$—phenoxy | $C_2H_5$ | H | H | 120—122 | Calc. | 56.5 | 3.6 | 3.0 |
| | | | | | | Found. | 56.3 | 3.7 | 3.0 |
| 69 | 2-Cl-4-$CF_3$—phenoxy | Sec-$C_4H_9$ | H | H | 112—115 | Calc. | 58.1 | 4.2 | 2.8 |
| | | | | | | Found. | 58.0 | 4.3 | 2.8 |
| 70 | $OCF_3$ | $CH_3$ | H | H | 100—101 | Calc. | 52.5 | 3.5 | 4.1 |
| | | | | | | Found. | 52.6 | 3.5 | 4.1 |
| 71 | $OCF_3$ | $C_2H_5$ | H | H | 123—125 | Calc. | 53.8 | 4.0 | 3.9 |
| | | | | | | Found. | 54.0 | 4.1 | 4.0 |

TABLE 3 (continued)

| Example No. | T | R | U | V | m.p. (°C) | | Analysis % C | H | N |
|---|---|---|---|---|---|---|---|---|---|
| 72 | $OCF_3$ | n-$C_3H_7$ | H | H | 132—134 | Calc.<br>Found. | 55.0<br>55.2 | 4.3<br>4.3 | 3.8<br>3.9 |
| 73 | $OCF_3$ | n-$C_4H_7$ | H | H | 123—124 | Calc.<br>Found. | 56.1<br>56.0 | 4.7<br>4.9 | 3.6<br>3.7 |
| 74 | $OCF_3$ | Sec-$C_4H_9$ | H | H | 98—99 | Calc.<br>Found. | 56.1<br>56.3 | 4.7<br>4.6 | 3.6<br>3.9 |
| 75 | $OCF_3$ | n-$C_5H_{11}$ | H | H | 114—116 | Calc.<br>Found. | 57.1<br>57.1 | 5.1<br>4.7 | 3.5<br>3.7 |
| 76 | $OCF_3$ | $—CH(CH_3)CH_2CH_2CH_3$ | H | H | 85—86 | Calc.<br>Found. | 57.1<br>57.2 | 5.1<br>5.1 | 3.5<br>3.7 |
| 77 | $CF_3$ | $CH_3$ | H | H | 164—166 | Calc.<br>Found. | 55.0<br>54.7 | 3.7<br>3.7 | 4.3<br>4.4 |
| 78 | $CF_3$ | $C_2H_5$ | H | H | 123—125 | Calc.<br>Found. | 56.3<br>56.2 | 4.1<br>4.1 | 4.1<br>4.2 |
| 79 | $CF_3$ | n-$C_3H_7$ | H | H | 129—132 | Calc.<br>Found. | 57.5<br>57.7 | 4.5<br>4.6 | 3.9<br>4.0 |

TABLE 3 (continued)

| Example No. | T | R | U | V | m.p. (°C) | | Analysis % C | H | N |
|---|---|---|---|---|---|---|---|---|---|
| 80 | $CF_3$ | i-$C_3H_7$ | H | H | 133—135 | Calc.<br>Found. | 57.5<br>57.3 | 4.5<br>4.6 | 3.9<br>4.0 |
| 81 | $CF_3$ | —$(CH_2)_2CH(CH_3)_2$ | H | H | 142—144 | Calc.<br>Found. | 59.5<br>59.4 | 5.2<br>5.3 | 3.7<br>3.8 |
| 82 | $CF_3$ | —$CH_2CF_3$ | H | H | 104—107 | Calc.<br>Found. | 48.6<br>48.5 | 2.8<br>2.8 | 3.5<br>3.4 |
| 83 | 2-Cl-4-$CF_3$-phenoxy | $CH_3$ | H | H | 132—134 | Calc.<br>Found. | 55.6<br>55.6 | 3.3<br>3.3 | 3.1<br>3.1 |
| 84 | 2-Cl-4-$CF_3$-phenoxy | n-$C_4H_9$ | H | H | 103—104 | Calc.<br>Found. | 58.1<br>58.3 | 4.2<br>4.4 | 2.8<br>2.9 |
| 85 | 2-Cl-4-$CF_3$-phenoxy | n-$C_5H_{11}$ | H | H | 98—100 | Calc.<br>Found. | 58.9<br>58.7 | 4.5<br>4.5 | 2.7<br>2.7 |
| 86 | 2-Cl-4-$CF_3$-phenoxy | —$CH(CH_3)CH_2CH_2CH_3$ | H | H | 89—91 | Calc.<br>Found. | 58.9<br>58.6 | 4.5<br>4.6 | 2.7<br>2.7 |
| 87 | 2-Cl-4-$CF_3$-phenoxy | —$CH_2CH_2CH(CH_3)_2$ | H | H | not isolated | Calc.<br>Found. | 58.9 | 4.5 | 2.7 |

TABLE 3 (continued)

| Example No. | T | R | U | V | m.p. (°C) | | Analysis % C | H | N |
|---|---|---|---|---|---|---|---|---|---|
| 88 | 2-Cl-4-$CF_3$-phenoxy | $—(CH_2)_9CH_3$ | H | H | 92—95 | Calc.<br>Found. | 62.1<br>62.2 | 5.7<br>5.9 | 2.4<br>2.3 |
| 89 | 2-Cl-4-$CF_3$-phenoxy | $—(CH_2)_{13}CH_3$ | H | H | 101—103 | Calc.<br>Found. | 64.2<br>64.6 | 6.5<br>6.3 | 2.2<br>2.1 |
| 90 | 2-Cl-4-$CF_3$-phenoxy | $—(CH_2)_{15}CH_3$ | H | H | 98—100 | Calc.<br>Found. | 65.1<br>65.0 | 6.8<br>7.1 | 2.1<br>2.0 |
| 91 | 2-Cl-4-$CF_3$-phenoxy | cyclohexyl | H | H | 138—140 | Calc.<br>Found. | 59.8<br>59.9 | 4.4<br>4.5 | 2.7<br>2.6 |
| 92 | 2-Cl-4-$CF_3$-phenoxy | benzyl | H | H | 114—116 | Calc.<br>Found. | 61.2<br>61.7 | 3.6<br>3.7 | 2.6<br>2.6 |
| 93 | 2-Cl-4-$CF_3$-phenoxy | phenyl | H | H | 128—130 | Calc.<br>Found. | 60.5<br>60.4 | 3.3<br>3.3 | 2.7<br>2.7 |
| 94 | 2-Cl-4-$CF_3$-phenoxy | $—CH(CH_3)CO_2C_2H_5$ | H | H | 109—111 | Calc.<br>Found. | 55.6<br>55.6 | 3.9<br>4.0 | 2.6<br>2.6 |
| 95 | 2-Cl-4-$CF_3$-phenoxy | $—CH_2CO_2C_2H_5$ | H | H | 119—121 | Calc.<br>Found. | 54.8<br>55.0 | 3.6<br>3.7 | 2.7<br>2.7 |

TABLE 3 (continued)

| Example No. | T | R | U | V | m.p. (°C) | | Analysis % C | H | N |
|---|---|---|---|---|---|---|---|---|---|
| 96 | 2-Cl-4-$CF_3$-phenoxy | 2-tetrahydrofurylmethyl | H | H | 124—126 | Calc.<br>Found. | 57.3<br>57.3 | 4.0<br>4.1 | 2.7<br>2.7 |
| 97 | Cl | n-$C_3H_7$ | H | H | 102—104 | Calc.<br>Found. | 59.7<br>59.8 | 5.0<br>4.9 | 4.4<br>4.4 |
| 98 | Cl | Sec-$C_4H_9$ | H | H | 90—93 | Calc.<br>Found. | 60.8<br>60.6 | 5.4<br>5.5 | 4.2<br>4.2 |
| 99 | 2-Cl-4-$CF_3$-phenoxy | $—CH_2CH_2OCH_3$ | H | H | 107—108 | Calc.<br>Found. | 55.5<br>55.6 | 3.8<br>3.9 | 2.8<br>2.9 |
| 100 | 2-Cl-4-$CF_3$-phenoxy | $—CH_2CH_2OC_2H_5$ | H | H | 103—104 | Calc.<br>Found. | 56.3<br>55.6 | 4.1<br>3.9 | 2.7<br>2.9 |
| 101 | 2-Cl-4-$CF_3$-phenoxy | $—(CH_2CH_2O)_2CH_3$ | H | H | 106—108 | Calc.<br>Found. | 55.4<br>55.5 | 4.2<br>4.3 | 2.6<br>2.6 |
| 102 | 2-Cl-4-$CF_3$-phenoxy | $—(CH_2CH_2O)_2C_2H_5$ | H | H | 86—88 | Calc.<br>Found. | 56.2<br>55.9 | 4.5<br>4.5 | 2.5<br>2.6 |
| 103 | 3-Cl-4-CH-phenoxy | n-$C_3H_7$ | Cl | Cl | 97—100 | Calc.<br>Found. | 54.4<br>54.4 | 3.3<br>3.3 | 5.5<br>5.1 |

TABLE 3 (continued)

| Example No. | T | R | U | V | m.p. (°C) | | Analysis % C | H | N |
|---|---|---|---|---|---|---|---|---|---|
| 104 | 3-Cl-4-CN-phenoxy | Sec-$C_4H_9$ | Cl | Cl | 103—105 | Calc.<br>Found. | 55.2<br>55.6 | 3.6<br>3.8 | 5.4<br>5.0 |
| 105 | 4-CN-phenoxy | n-$C_3H_7$ | Cl | H | 68—70 | Calc.<br>Found. | 62.9<br>62.1 | 4.3<br>4.4 | 6.4<br>6.1 |
| 106 | 4-CN-phenoxy | Sec-$C_4H_9$ | Cl | H | 77—79 | Calc.<br>Found. | 63.7<br>62.9 | 4.6<br>4.8 | 6.2<br>6.0 |
| 107 | 4-$NO_2$-phenoxy | n-$C_3H_7$ | Cl | Cl | 85—87 | Calc.<br>Found. | 53.5<br>53.6 | 3.7<br>3.7 | 5.7<br>5.8 |
| 108 | 3,5-dichloro-2-pyridyloxy | Sec-$C_4H_7$ | C | H | 116—118 | Calc.<br>Found. | 53.1<br>53.0 | 3.8<br>3.56 | 5.6<br>5.6 |
| 109 | 3,5-dichloro-2-pyridyloxy | n-$C_3H_7$ | Cl | H | 141—143 | Calc.<br>Found. | 52.1<br>52.0 | 3.5<br>3.4 | 5.8<br>5.7 |
| 110 | 3,5-dichloro-2-pyridyloxy | Sec-$C_4H_9$ | Cl | Cl | 78—80 | Calc.<br>Found. | 49.6<br>50.5 | 3.4<br>3.3 | 5.3<br>5.0 |
| 111 | 3,5-dichloro-2-pyridyloxy | n-$C_3H_7$ | Cl | Cl | 153—155 | Calc.<br>Found. | 48.6<br>47.8 | 3.1<br>3.0 | 5.4<br>5.3 |
| 112 | 4-$NO_2$-phenoxy | Sec-$C_4H_9$ | Cl | Cl | 131—133 | Calc.<br>Found. | 54.4<br>54.5 | 3.9<br>3.9 | 5.5<br>5.5 |
| 113 | 4-$NO_2$-phenoxy | n-$C_5H_{11}$ | Cl | Cl | 132—134 | Calc.<br>Found. | 55.3<br>55.3 | 4.2<br>4.1 | 5.4<br>5.4 |
| 114 | 4-$NO_2$-phenoxy | n-$C_4H_9$ | Cl | Cl | 129—130 | Calc.<br>Found. | 54.4<br>54.9 | 3.9<br>4.0 | 5.5<br>5.5 |

**Claims for the Contracting States: BE CH DE FR GB IT LI LU NL SE**

1. A benzoylurea compound of formula:—

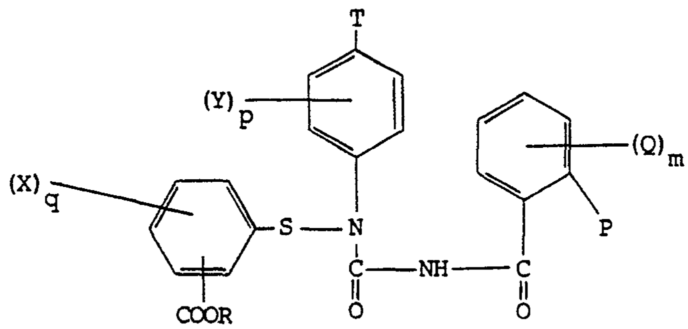

(I)

—

in which each of P and Q independently represents a halogen atom or an alkyl group; m represents 0, 1 or 2; R represents an optionally-substituted alkyl, alkenyl, alkynyl, cycloalkyl or phenyl group, a hydrogen atom, one equivalent of an alkali metal or alkaline earth metal, an ammonium or substituted ammonium group, or a group of general formula $—(CH_2CH_2O)_nR^1$ in which $R^1$ represents an optionally substituted alkyl, alkenyl, alkynyl, cycloalkyl or phenyl group, and n represents 1, 2, 3, 4 or 5; each X independently represents a halogen atom, a cyano, nitro or carboxy group, or an alkyl, alkoxy, alkylthio, cycloalkyl, cycloalkyloxy, cycloalkylthio, alkenyl, alkenylthio, alkylcarbonyl, alkoxycarbonyl, alkylsulphonyl, alkynyl, phenyl, phenoxy, phenylthio or amino group; q = 0, 1, 2, 3 or 4; T represents a halogen atom, an alkyl, haloalkyl, alkoxy, haloalkoxy, alkenyloxy or haloalkenyloxy group, or a phenoxy or pyridyloxy group optionally substituted by one or more substituents selected from halogen atoms and nitro, alkyl, haloalkyl and cyano groups; each Y independently represents a halogen atom or a nitro, cyano, alkyl or haloalkyl group; and p = 0, 1, 2, 3 or 4; the optional substituents in an optionally substituted alkyl, alkenyl or alkynyl moiety being selected from halogen atoms; alkoxy, haloalkoxy, hydroxy, carboxy, alkoxycarbonyl and cycloalkyl groups; saturated or unsaturated heterocyclic groups containing one oxygen, sulphur or nitrogen atom, and having 5 to 6 atoms in the ring; groups of formula $—O—(CH_2CH_2O)_n$ alkyl when n is 1 to 4; and optionally substituted phenyl groups; the optional substituents in an optionally substituted cycloalkyl moiety being selected from alkyl and haloalkyl groups and those substituent groups listed in respect of alkyl; and the optional substituents in an optionally substituted phenyl group being selected from halogen atoms and alkyl, haloalkyl, alkoxy, haloalkoxy, alkenyl, haloalkenyl, nitro and cyano groups; an alkyl, alkenyl or alkynyl moiety having up to 6 carbon atoms; a cycloalkyl group having 3 to 7 ring carbon atoms; except that when R represents an optionally substituted alkyl group the alkyl moiety may contain 1 to 20 carbon atoms, and when $R^1$ represents an alkyl or haloalkyl group, the halogen being chlorine or fluorine, the alkyl or haloalkyl group may contain 1 to 10 carbon atoms.

2. A compound as claimed in claim 1, in which each of P and Q independently represents a C(1—4) alkyl group or a fluorine, chlorine or bromine atom, and m is 0 or 1.

3. A compound as claimed in either claim 1 or claim 2, in which T represents a bromine, fluorine or chlorine atom; a C(1—4) alkyl, haloalkyl or haloalkoxy group; an optionally substituted phenoxy group in which the optional substituents are selected from C(1—4) alkyl and haloalkyl group, fluorine and chlorine atoms, and nitro and cyano groups; or an optionally substituted pyridyloxy group in which the optional substituents are selected from trifluoromethyl groups and fluorine and chlorine atoms.

4. A compound as claimed in any one of claims 1 to 3, in which T represents an optionally substituted 2-pyridyloxy group in which the optional substituents are selected from halogen atoms and haloalkyl groups, or T represents a 2-chloro-4-trifluoromethylphenoxy, 2-fluoro-4-trifluoromethylphenoxy or 2-cyano-4-trifluoromethylphenoxy group.

5. A compound as claimed in any one of claims 1 to 4 in which Y represents a fluorine, chlorine or bromine atom, a C(1—4) alkyl or haloalkyl group, or a nitro or cyano group, and p is 0, 1 or 2.

6. A compound as claimed in any one of claims 1 to 5, in which X represents a bromine, chlorine or fluorine atom or a C(1—4) alkyl group, and q is 0 or 1.

7. A compound as claimed in any one of claims 1 to 6, in which R represents an optionally substituted alkyl group containing 1 to 20 carbon atoms, a C(3—6) cycloalkyl group, or an optionally substituted phenyl group, the substituents in the optionally substituted phenyl group being selected from C(1—4) alkyl and haloalkyl groups, nitro and cyano groups, and halogen atoms, and the substituents in an optionally substituted alkyl group being selected from halogen atoms, C(1—4) alkoxy groups, carboxy groups, alkoxycarbonyl groups containing 2 to 5 carbon atoms, groups of formula $—O—(CH_2CH_2O)_n$ alkyl where n is 1 to 4, and the alkyl moiety has 1 to 4 carbon atoms, optionally substituted phenyl groups in which the optional substituents are as given above for an optionally substituted phenyl group R, and saturated or unsaturated heterocyclic groups containing one oxygen, sulphur or nitrogen atom, and having 5 or 6 atoms in the ring.

8. A compound as claimed in claim 7, in which R represents an unsubstituted alkyl group having from 1 to 10 carbon atoms.

9. A compound as claimed in any one of claims 1 to 6, in which $R^1$ represents an alkyl or haloalkyl group containing 1 to 10 carbon atoms, the halogen being chlorine or fluorine, and n is 1 or 2.

10. A compound as claimed in any one of claims 1 to 9, in which the —COOR group is located in the 2-position of the phenyl group to which it is attached.

11. A process for preparing a compound as claimed in any one of claims 1 to 10, which comprises reacting a benzoylisocyanate of formula:

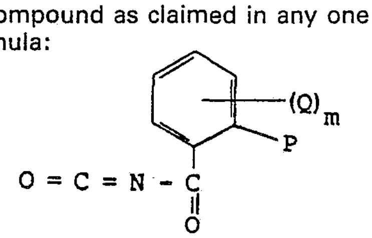

(III)

with a sulphenamide of formula:

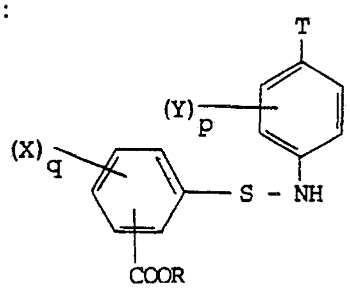

(IV)

in which P, Q, m, R, X, q, T, Y and p have the meanings given in any one of claims 1 to 10.

12. A process as claimed in claim 11, in which the reaction temperature is in the range of from 0 to 100°C.

13. A pesticidal composition which comprises a compound as claimed in any one of claims 1 to 10, together with a carrier.

14. A composition as claimed in claim 13, which comprises at least two carriers, at least one of which is a surface-active agent.

15. A method of combating pests at a locus, which comprises applying an insecticidally effective amount of a compound as claimed in any one of claims 1 to 10 or a composition as claimed in either claim 13 or claim 14 to the locus.

**Claims for the Contracting State: AT**

1. A process for the preparation of a benzoylurea compound of formula:—

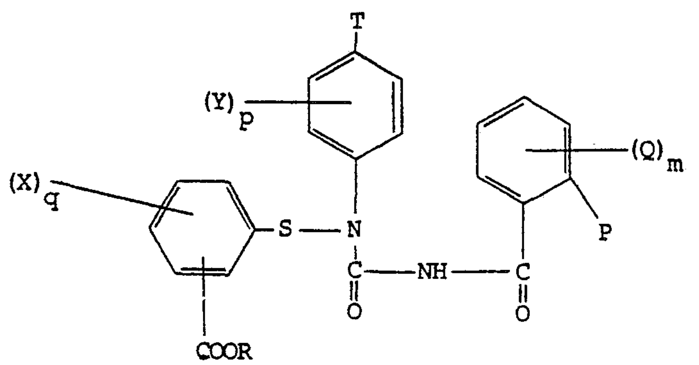

(I)

in which each of P and Q independently represents a halogen atom or an alkyl group; m represents 0, 1 or 2; R represents an optionally-substituted alkyl, alkenyl, alkynyl, cycloalkyl or phenyl group, a hydrogen atom, one equivalent of an alkali metal or alkaline earth metal, an ammonium or substituted ammonium group, or a group of general formula —$(CH_2CH_2O)_nR^1$ in which $R^1$ represents an optionally substituted alkyl, alkenyl, alkynyl, cycloalkyl or phenyl group, and n represents 1, 2, 3, 4 or 5; each X independently represents a halogen atom, a cyano, nitro or carboxy group, or an alkyl, alkoxy, alkylthio, cycloalkyl, cycloalkyloxy, cycloalkylthio, alkenyl, alkenylthio, alkylcarbonyl, alkoxycarbonyl, alkylsulphonyl, alkynyl, phenyl, phenoxy, phenylthio or amino group; q = 0, 1, 2, 3 or 4; T represents a halogen atom, an alkyl, haloalkyl, alkoxy, haloalkoxy, alkenyloxy or haloalkenyloxy group, or a phenoxy or pyridyloxy group optionally substituted by one or more substituents selected from halogen atoms and nitro, alkyl, haloalkyl and cyano groups; each Y independently represents a halogen atom or a nitro, cyano, alkyl or haloalkyl group; and p = 0, 1, 2, 3 or 4; the optional substituents in an optionally substituted alkyl, alkenyl or alkynyl moiety being selected from halogen atoms; alkoxy, haloalkoxy, hydroxy, carboxy, alkoxycarbonyl and

cycloalkyl groups; saturated or unsaturated heterocyclic groups containing one oxygen, sulphur or nitrogen atom, and having 5 to 6 atoms in the ring; groups of formula —O—$(CH_2CH_2O)_n$ alkyl when n is 1 to 4; and optionally substituted phenyl groups; the optional substituents in an optionally substituted cycloalkyl moiety being selected from alkyl and haloalkyl groups and those substituent groups listed in respect of alkyl; and the optional substituents in an optionally substituted phenyl group being selected from halogen atoms and alkyl, haloalkyl, alkoxy, haloalkoxy, alkenyl, haloalkenyl, nitro and cyano groups; an alkyl, alkenyl or alkynyl moiety having up to 6 carbon atoms; a cycloalkyl group having 3 to 7 ring carbon atoms; except that when R represents an optionally substituted alkyl group the alkyl moiety may contain 1 to 20 carbon atoms, and when $R^1$ represents an alkyl or haloalkyl group, the halogen being chlorine or fluorine, the alkyl or haloalkyl group may contain 1 to 10 carbon atoms, which comprises reacting a benzoylisocyanate of formula:

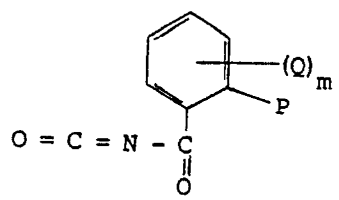

(III)

with a sulphenamide of formula:

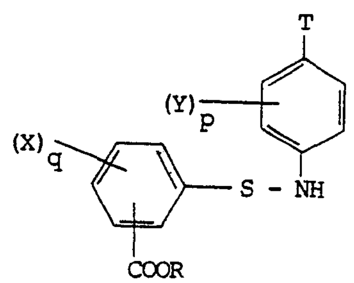

(IV)

in which P, Q, m, R, X, q, T, Y and p have the meanings given above.

2. A process as claimed in claim 1, in which each of P and Q independently represents a C(1—4) alkyl group or a fluorine, chlorine or bromine atom, and m is 0 or 1.

3. A process as claimed in either claim 1 or claim 2, in which T represents a bromine, fluorine or chlorine atom; a C(1—4) alkyl, haloalkyl or haloalkoxy group; an optionally substituted phenoxy group in which the optional substituents are selected from C(1—4) alkyl and haloalkyl group, fluorine and chlorine atoms, and nitro and cyano groups; or an optionally substituted pyridyloxy group in which the optional substituents are selected from trifluoromethyl groups and fluorine and chlorine atoms.

4. A process as claimed in any one of claims 1 to 3, in which T represents an optionally substituted 2-pyridyloxy group in which the optional substituents are selected from halogen atoms and haloalkyl groups, or T represents a 2-chloro-4-trifluoromethylphenoxy, 2-fluoro-4-trifluoromethylphenoxy or 2-cyano-4-trifluoromethylphenoxy group.

5. A process as claimed in any one of claims 1 to 4 in which Y represents a fluorine, chlorine or bromine atom, a C(1—4) alkyl or haloalkyl group, or a nitro or cyano group, and p is 0, 1 or 2.

6. A process as claimed in any one of claims 1 to 5, in which X represents a bromine, chlorine or fluorine atom or a C(1—4) alkyl group, and q is 0 or 1.

7. A process as claimed in any one of claims 1 to 6, in which R represents an optionally substituted alkyl group containing 1 to 20 carbon atoms, a C(3—6) cycloalkyl group, or an optionally substituted phenyl group, the substituents in the optionally substituted phenyl group being selected from C(1—4) alkyl and haloalkyl groups, nitro and cyano groups, and halogen atoms, and the substituents in an optionally substituted alkyl group being selected from halogen atoms, C(1—4) alkoxy groups, carboxy groups, alkoxycarbonyl groups containing 2 to 5 carbon atoms, groups of formula —O—$(CH_2CHO)_n$ alkyl where n is 1 to 4, and the alkyl moiety has 1 to 4 carbon atoms, optionally substituted phenyl groups in which the optional substituents are as given above for an optionally substituted phenyl group R, and saturated or unsaturated heterocyclic groups containing one oxygen, sulphur or nitrogen atom, and having 5 or 6 atoms in the ring.

8. A process as claimed in claim 7, in which R represents an unsubstituted alkyl group having from 1 to 10 carbon atoms.

9. A process as claimed in any one of claims 1 to 6, in which $R^1$ represents an alkyl or haloalkyl group containing 1 to 10 carbon atoms, the halogen being chlorine or fluorine, and n is 1 or 2.

10. A process as claimed in any one of claims 1 to 9, in which the —COOR group is located in the 2-position of the phenyl group to which it is attached.

11. A process as claimed in any of claims 1 to 10, in which the reaction temperature is in the range of from 0 to 100°C.

12. A pesticidal composition which comprises a compound of formula I as defined in any one of claims 1 to 10, together with a carrier.

13. A composition as claimed in claim 12, which comprises at least two carriers, at least one of which is a surface-active agent.

14. A method of combating pests at a locus, which comprises applying an insecticidally effective amount of a compound of formula I as defined in any one of claims 1 to 10 or a composition as claimed in either claim 12 or claim 13 to the locus.

**Patentansprüche für die Vertragsstaaten: BE CH DE FR GB IT LI LU NL SE**

1. Benzoylharnstoffverbindung der Formel:

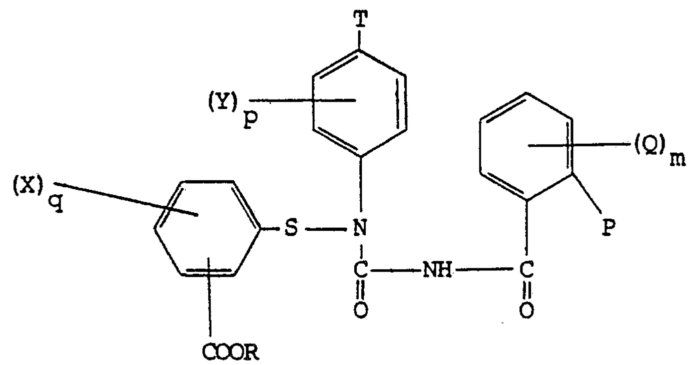

(I)

worin jeder Rest P und Q unabhängig voneinander ein Halogenatom oder eine Alkylgruppe bedeutet; m den Wert 0, 1 oder 2 aufweist; R eine gegebenenfalls substituierte Alkyl-, Alkenyl-, Alkinyl-, Cycloalkyl- oder Phenylgruppe, ein Wasserstoffatom, ein Äquivalent eines Alkalimetalles oder Erdalkalimetalles, eine Ammoniumgruppe oder substituierte Ammoniumgruppe oder eine Gruppe der allgemeinen Formel $—(CH_2CH_2O)_nR^1$ bedeutet, worin $R^1$ eine gegebenenfalls substituierte Alkyl-, Alkenyl-, Alkinyl-, Cycloalkyl- oder Phenylgruppe darstellt und n den Wert 1, 2, 3, 4 oder 5 aufweist; jeder Rest X unabhängig voneinander ein Halogenatom, ein Cyano-, Nitro oder Carboxygruppe oder eine Alkyl-, Alkoxy-, Alkylthio-, Cycloalkyl-, Cycloalkyloxy-, Cycloalkylthio-, Alkenyl-, Alkenylthio-, Alkylcarbonyl-, Alkoxycarbonyl-, Alkylsulfonyl-, Alkinyl-, Phenyl-, Phenoxy-, Phenylthio- oder Aminogruppe darstellt; q den Wert 0, 1, 2, 3 oder 4 aufweist; T ein Halogenatom, eine Alkyl-, Halogenalkyl-, Alkoxy-, Halogenalkoxy-, Alkenyloxy- oder Halogenalkenyloxygruppe darstellt oder eine Phenoxy- oder Pyridyloxygruppe bedeutet, die gegebenenfalls durch einen oder mehrere Substituenten, ausgewählt unter Halogenatomen und Nitro-, Alkyl-, Halogenalkyl- und Cyanogruppen, substituiert sind; jeder Rest Y unabhängig voneinander ein Halogenatom oder eine Nitro-, Cyano-, Alkyl- oder Halogenalkylgruppe darstellt; und p den Wert 0, 1, 2, 3 oder 4 aufweist; wobei die fakultativen Substituenten in einem gegebenenfalls substituierten Alkyl-, Alkenyl- oder Alkinylrest ausgewählt sind unter Halogenatomen; Alkoxy-, Halogenalkoxy-, Hydroxy-, Carboxy-, Alkoxycarbonyl- und Cycloalkylgruppen; gesättigten oder ungesättigten heterocyclischen Gruppen mit einem Sauerstoff-, Schwefel- oder Stickstoffatom und 5 oder 6 Ringatomen; Gruppen der Formel $—O—(CH_2CH_2O)_n$ alkyl, worin n den Wert 1 bis 4 aufweist; und gegebenenfalls substituierten Phenylgruppen; wobei die fakultativen Substituenten in einem gegebenenfalls substituierten Cycloalkylrest ausgewählt sind unter Alkyl- und Halogenalkylgruppen und jenen Substituentengruppen, die im Zusammenhang mit Alkyl angegeben sind; und wobei die fakulativen Substituenten in einer gegebenenfalls substituierten Phenylgruppe ausgewählt sind unter Halogenatomen und Alkyl-, Halogenalkyl-, Alkoxy-, Halogenalkoxy-, Alkenyl-, Halogenalkenyl-, Nitro- und Cyanogruppen; wobei ein Alkyl-, Alkenyl- oder Alkinylrest bis zu 6 Kohlenstoffatome aufweist; wobei eine Cycloalkylgruppe 3 bis 7 Ringkohlenstoffatome aufweist; mit der Ausnahme, daß dann, wenn R eine gegebenenfalls substiuierte Alkylgruppe bedeutet, der Alkylrest 1 bis 20 Kohlenstoffatome enthalten kann, und daß dann, wenn $R^1$ eine Alkyl- oder Halogenalkylgruppe darstellt, wobei das Halogen Chlor oder Fluor ist, die Alkyl- oder Halogenalkylgruppe 1 bis 10 Kohlenstoffatome enthalten kann.

2. Verbindung nach Anspruch 1, worin jeder der Reste P und Q unabhängig voneinander eine C(1—4)-alkylgruppe oder ein Fluor-, Chlor- oder Bromatom bedeutet und m den Wert 0 oder 1 aufweist.

3. Verbindung nach Anspruch 1 oder 2, worin T ein Brom-, Fluor- oder Chloratom; eine C(1—4)alkyl-, -halogenalkyl- oder -halogenalkoxygruppe; eine gegebenenfalls substituierte Phenoxygruppe, worin die fakultativen Substituenten ausgewählt sind unter C(1—4)alkyl- under -halogenalkylgruppen, Fluor- und Chloratomen und Nitro- und Cyanogruppen; oder eine gegebenenfalls substituierte Pyridyloxygruppe bedeutet, worin die fakultativen Substituenten ausgewählt sind unter Trifluormethylgruppen und Fluor- und Chloratomen.

4. Verbindung nach einem der Ansprüche 1 bis 3, worin T eine gegebenenfalls substituierte 2-Pyridyloxygruppe darstellt, worin die fakultativen Substituenten ausgewählt sind unter Halogenatomen und Halogenalkylgruppen, oder T eine 2-Chlor-4-trifluoromethylphenoxy-, 2-Fluor-4-trifluormethylphenoxy- oder 2-Cyano-4-trifluormethylphenoxygruppe bedeutet.

5. Verbindung nach einem der Ansprüche 1 bis 4, worin Y ein Fluor-, Chlor- oder Bromatom, eine C(1—4)alkyl- oder -halogenalkylgruppe oder eine Nitro- oder Cyanogruppe darstellt und p den Wert 0, 1 oder 2 aufweist.

6. Verbindung nach einem der Ansprüche 1 bis 5, worin X ein Brom-, Chlor- oder Fluoratom oder eine C(1—4)alkylgruppe bedeutet und q den Wert 0 oder 1 aufweist.

7. Verbindung nach einem der Ansprüche 1 bis 6, worin R eine gegebenenfalls substituierte Alkylgruppe mit 1 bis 20 Kohlenstoffatomen, eine C(3—6)cycloalkylgruppe oder eine gegebenenfalls substituierte Phenylgruppe darstellt, wobei die Substituenten in der gegebenenfalls substituierten Phenylgruppe ausgewählt sind unter C(1—4)alkyl- und -halogenalkylgruppen, Nitro- und Cyanogruppen und Halogenatomen, und wobei die Substituenten in einer gegebenenfalls substituierten Alkylgruppe ausgewählt sind unter Halogenatomen, C(1—4)alkoxygruppen, Carboxygruppen, Alkoxycarbonylgruppen mit 2 bis 5 Kohlenstoffatomen, Gruppen der Formel $—O—(CH_2CH_2O)_n$alkyl, worin n den Wert 1 bis 4 aufweist und der Alkylrest 1 bis 4 Kohlenstoffatome hat, gegebenenfalls substituierten Phenylgruppen, worin die fakultativen Substituenten die wie zuvor für eine gegebenenfalls substituierte Phenylgruppe R angegebenen sind, und gesättigten oder ungesättigten heterocyclischen Gruppen mit einem Sauerstoff-, Schwefel- oder Stickstoffatom mit 5 bis 6 Ringatomen.

8. Verbindung nach Anspruch 7, worin R eine unsubstituierte Alkylgruppe mit 1 bis 10 Kohlenstoffatomen bedeutet.

9. Verbindung nach einem der Ansprüche 1 bis 6, worin $R^1$ eine Alkyl- oder Halogenalkylgruppe mit 1 bis 10 Kohlenstoffatomen bedeutet, worin das Halogen Chlor oder Fluor ist, und n den Wert 1 oder 2 aufweist.

10. Verbindung nach einem der Ansprüche 1 bis 9, worin die —COOR-Gruppe in der 2-Stellung des Phenylringes angeordnet ist, an den sie gebunden ist.

11. Verfahren zur Herstellung einer Verbindung gemäß einem der Ansprüche 1 bis 10, welches ein Umsetzen eines Benzoylisocyanats der Formel:

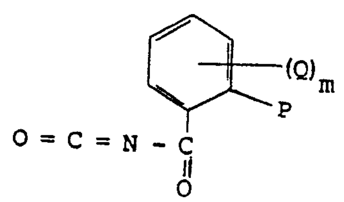

(III)

mit einem Sulfenamid der Formel:

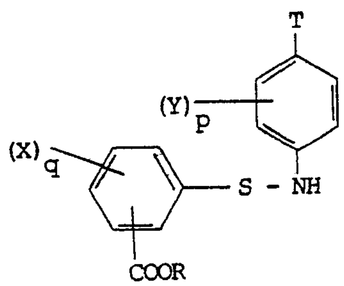

(IV)

worin P, Q, m, R, X, q, T, Y und p die in einem der Ansprüche 1 bis 10 angegebenen Bedeutungen aufweisen, umfaßt.

12. Verfahren nach Anspruch 11, worin die Umsetzungstemperatur im Bereich von 0 bis 100°C liegt.

13. Pestizide Zusammensetzung, welche eine Verbindung nach einem der Ansprüche 1 bis 10 zusammen mit einem Träger umfaßt.

14. Zusammensetzung nach Anspruch 13, welche wenigstens zwei Träger umfaßt, von denen wenigstens einer ein oberflächenaktives Mittel ist.

15. Verfahren zur Bekämpfung von Schädlingen an einem Ort, welches ein Aufbringen einer insektizid wirksamen Menge einer Verbindung nach einem der Ansprüche 1 bis 10 oder einer Zusammensetzung nach anspruch 13 oder 14 auf den Ort umfaßt.

**Patentansprüche für den Vertragsstaat: AT**

1. Verfahren zur Herstellung einer Benzoylharnstoffverbindung der Formel:

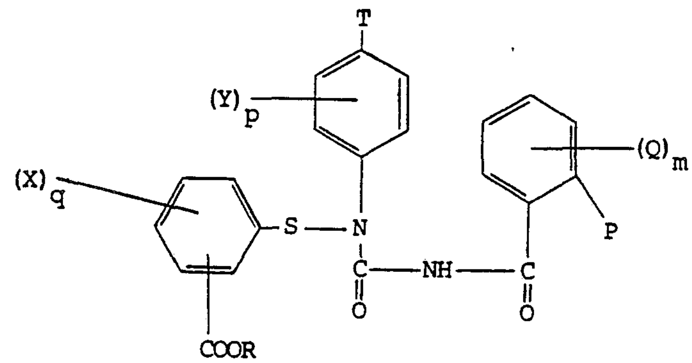

(I)

worin jeder Rest P und Q unabhängig voneinander ein Halogenatom oder eine Alkylgruppe bedeutet; m den Wert 0, 1 oder 2 aufweist; R eine gegebenenfalls substituierte Alkyl-, Alkenyl-, Alkinyl-, Cycloalkyl- oder Phenylgruppe, ein Wasserstoffatom, ein Äquivalent eines Alkalimetalles oder Erdalkalimetalles, eine Ammoniumgruppe oder substituierte Ammoniumgruppe oder eine Gruppe der allgemeinen Formel $—(CH_2CH_2O)_nR^1$ bedeutet, worin $R^1$ eine gegebenenfalls substituierte Alkyl-, Alkenyl-, Alkinyl-, Cycloalkyl- oder Phenylgruppe darstellt und n den Wert 1, 2, 3, 4 oder 5 aufweist; jeder Rest X unabhängig voneinander ein Halogenatom, ein Cyano-, Nitro oder Carboxygruppe oder eine Alkyl-, Alkoxy-, Alkylthio-, Cycloalkyl-, Cycloalkyloxy-, Cycloalkylthio-, Alkenyl-, Alkenylthio-, Alkylcarbonyl-, Alkoxycarbonyl-, Alkylsulfonyl-, Alkinyl-, Phenyl-, Phenoxy-, Phenylthio- oder Aminogruppe darstellt; q den Wert 0, 1, 2, 3 oder 4 aufweist; T ein Halogenatom, eine Alkyl-, Halogenalkyl-, Alkoxy-, Halogenalkoxy-, Alkenyloxy- oder Halogenalkenyloxygruppe darstellt oder eine Phenoxy- oder Pyridyloxygruppe bedeutet, die gegebenenfalls durch einen oder mehrere Substituenten, ausgewählt unter Halogenatomen und Nitro-, Alkyl-, Halogenalkyl- und Cyanogruppen, substituiert sind; jeder Rest Y unabhängig voneinander ein Halogenatom oder eine Nitro-, Cyano-, Alkyl- oder Halogenalkylgruppe darstellt; und p den Wert 0, 1, 2, 3 oder 4 aufweist; wobei die fakultativen Substituenten in einem gegebenenfalls substituierten Alkyl-, Alkenyl- oder Alkinylrest ausgewählt sind unter Halogenatomen; Alkoxy-, Halogenalkoxy-, Hydroxy-, Carboxy-, Alkoxycarbonyl- und Cycloalkylgruppen; gesättigten oder ungesättigten heterocyclischen Gruppen mit einem Sauerstoff-, Schwefel- oder Stickstoffatom und 5 oder 6 Ringatomen; Gruppen der Formel $—O—(CH_2CH_2O)_n$alkyl, worin n den Wert 1 bis 4 aufweist; und gegebenenfalls substituierten Phenylgruppen; wobei die fakultativen Substituenten in enem gegebenenfalls substituierten Cycloalkylrest ausgewählt sind unter Alkyl- und Halogenalkylgruppen und jenen Substituentengruppen, die im Zusammenhang mit Alkyl angegeben sind; und wobei die fakulativen Substituenten in einer gegebenenfalls substituierten Phenylgruppe ausgewählt sind unter Halogenatomen und Alkyl-, Halogenalkyl-, Alkoxy-, Halogenalkoxy-, Alkenyl-, Halogenalkenyl-, Nitro- und Cyanogruppen; wobei ein Alkyl-, Alkenyl- oder Alkinylrest bis zu 6 Kohlenstoffatome aufweist; wobei eine Cycloalkylgruppe 3 bis 7 Ringkohlenstoffatome aufweist; mit der Ausnahme, daß dann, wenn R eine gegebenenfalls substiuierte Alkylgruppe bedeutet, der Alkylrest 1 bis 20 Kohlenstoffatome enthalten kann, und daß dann, wenn $R^1$ eine Alkyl- oder Halogenalkylgruppe darstellt, wobei das Halogen Chlor oder Fluor ist, die Alkyl- oder Halogenalkylgruppe 1 bis 10 Kohlenstoffatome enthalten kann, welches ein Umsetzen eines Benzoylisocyanats der Formel:

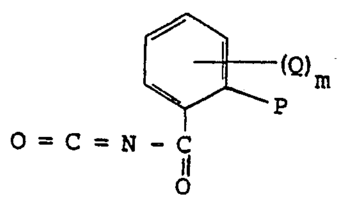

(III)

mit einem Sulfenamid der Formel:

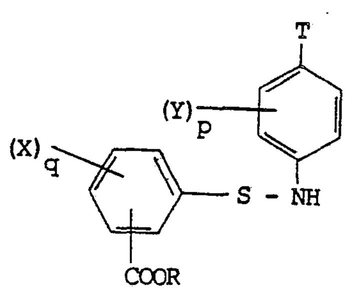

(IV)

worin P, Q, m, R, X, q, T, Y und p die vorstehend angegebenen Bedeutungen aufweisen umfaßt.

2. Verfahren nach Anspruch 1, worin jeder der Reste P und Q unabhängig voneinander eine C(1—4)-alkylgruppe oder ein Fluor-, Chlor- oder Bromatom bedeutet und m den Wert 0 oder 1 aufweist.

3. Verfahren nach Anspruch 1 oder 2, worin T ein Brom-, Fluor- oder Chloratom; eine C(1—4)alkyl-, -halogenalkyl- oder -halogenalkoxygruppe; eine gegebenenfalls substituierte Phenoxygruppe, worin die fakultativen Substituenten ausgewählt sind unter C(1—4)alkyl- und -halogenalkylgruppen, Fluor- und Chloratomen und Nitro- und Cyanogruppen; oder eine gegebenenfalls substituierte Pyridylgruppe bedeutet, worin die fakultativen Substituenten ausgewählt sind unter Trifluormethylgruppen und Fluor- und Chloratomen.

4. Verfahren nach einem der Ansprüche 1 bis 3, worin T eine gegebenenfalls substituierte 2-Pyridyloxygruppe darstellt, worin die fakultativen Substituenten ausgewählt sind unter Halogenatomen und Halogenalkylgruppen, oder T eine 2-Chlor-4-trifluormethylphenoxy-, 2-Fluor-4-trifluormethylphenoxy- oder 2-Cyano-4-trifluormethylphenoxy- oder 2-Cyano-4-trifluormethylphenoxygruppe bedeutet.

5. Verfahren nach einem der Ansprüche 1 bis 4, worin Y ein Fluor-, Chlor- oder Bromatom, eine C(1—4)-alkyl- oder -halogenalkylgruppe oder eine Nitro- oder Cyanogruppe darstellt und p den Wert 0, 1 oder 2 aufweist.

6. Verfahren nach einem der Ansprüche 1 bis 5, worin X ein Brom-, Chlor- oder Fluoratom oder eine C(1—4)alkylgruppe bedeutet und q den Wert 0 oder 1 aufweist.

7. Verfahren nach einem der Ansprüche 1 bis 6, worin R eine gegebenenfalls substituierte Alkylgruppe mit 1 bis 20 Kohlenstoffatomen, eine C(3—6)cycloalkylgruppe oder eine gegebenenfalls substituierte Phenylgruppe darstellt, wobei die Substituenten in der gegebenenfalls substituierten Phenylgruppe ausgewählt sind unter C(1—4)alkyl- und -halogenalkylgruppen, Nitro- und Cyanogruppen und Halogenatomen, und wobei die Substituenten in einer gegebenenfalls substituierten Alkylgruppe ausgewählt sind unter Halogenatomen, C(1—4)alkoxygruppen, Carboxygruppen, Alkoxycarbonylgruppen mit 2 bis 5 Kohlenstoffatomen, Gruppen der Formel $—O—(CH_2CH_2O)_n$alkyl, worin n den Wert 1 bis 4 aufweist und der Alkylrest 1 bis 4 Kohlenstoffatome hat, gegebenenfalls substituierten Phenylgruppen, worin die fakultativen Substituenten die wie zuvor für eine gegebenenfalls substituierte Phenylgruppe R angegebenen sind, und gesättigten oder ungesättigten heterocyclischen Gruppen mit einem Sauerstoff-, Schwefel- oder Stickstoffatom mit 5 bis 6 Ringatomen.

8. Verfahren nach Anspruch 7, worin R eine unsubstituierte Alkylgruppe mit 1 bis 10 Kohlenstoffatomen bedeutet.

9. Verfahren nach einem der Ansprüche 1 bis 6, worin $R^1$ eine Alkyl- oder Halogenalkylgruppe mit 1 bis 10 Kohlenstoffatomen bedeutet, worin das Halogen Chlor oder Fluor ist, und n den Wert 1 oder 2 aufweist.

10. Verfahren nach einem der Ansprüche 1 bis 9, worin die —COOR-Gruppe in der 2-Stellung des Phenylringes angeordnet ist, an den sie gebunden ist.

11. Verfahren nach einem der Ansprüche 1 bis 10, worin die Umsetzungstemperatur im Bereich von 0 bis 100°C liegt.

12. Pestizide Zusammensetzung, welche eine Verbindung der Formel I, wie in einem der Ansprüche 1 bis 10 definiert, zusammen mit einem Träger umfaßt.

13. Zusammensetzung nach Anspruch 12, welche wenigstens 2 Träger umfaßt, von denen wenigstens einer ein oberflächenaktives Mittel ist.

14. Verfahren zur Bekämpfung von Schädlingen an einem Ort, welches ein Aufbringen einer insektizid wirksamen Menge einer Verbindung der Formel I, wie in einem der Ansprüche 1 bis 10 definiert, oder einer Zusammensetzung, wie in Anspruch 12 oder 13 definiert, auf den Ort umfaßt.

**Revendications pour les Etats Contractants: BE CH DE FR GB IT LU NL SE LI**

1. Un dérivé de benzoylurée de formule

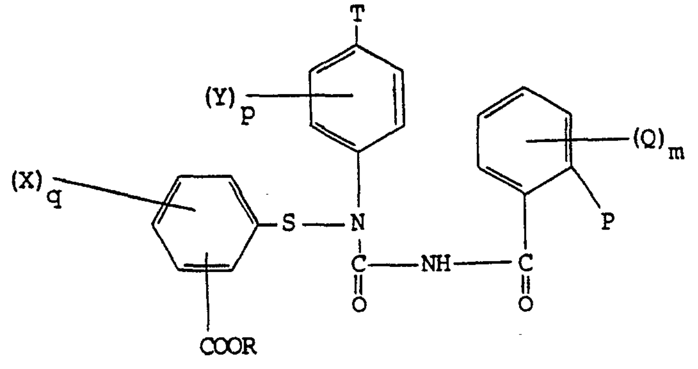

(I)

où P et Q représentent chacun indépendamment un atome d'halogène ou un groupe alcoyle; m représente 0, 1 ou 2; R représente un groupe alcoyle, alcényle, alcynyle, cycloalcoyle ou phényle éventuellement

substitué, un atome d'hydrogène, un équivalent d'un métal alcalin ou d'un métal alcalino-terreux, un groupe ammonium ou ammonium substitué ou un groupe de formule générale $-(CH_2CH_2O)_nR^1$ où $R^1$ représente un groupe alcoyle, alcényle, alcynyle, cycloalcoyle ou phényle éventuellement substitué, et n représente 1, 2, 3, 4 ou 5; chaque X indépendamment représente un atome d'halogène, un groupe cyano, nitro ou carboxy ou un groupe alcoyle, alcoxy, alcoylthio, cycloalcoyle, cycloalcoyloxy, cycloalcoylthio, alcényle, alcénylthio, alcoylcarbonyle, alcoxycarbonyle, alcoylsulfonyle, alcynyle, phényle, phénoxy, phénylthio ou amino; q = 0, 1, 2, 3 ou 4; T représente un atome d'halogène, un groupe alcoyle, haloalcoyle, alcoxy, haloalcoxy, alcényloxy ou haloalcényloxy ou un groupe phénoxy ou pyridyloxy éventuellement substitué par un ou plusieurs substituants choisi parmi des atomes d'halogènes et des groupes nitro, alcoyle, haloalcoyle et cyano; chaque Y indépendamment représente un atome d'halogène ou un groupe nitro, cyano, alcoyle ou haloalcoyle; et p = 0, 1, 2, 3 ou 4; les substituants éventuels dans une portion alcoyle, alcényle ou alcynyle éventuellement substituée étant choisis parmi des atomes d'halogènes; des groupes alcoxy, haloalcoxy, hydroxy, carboxy, alcoxycarbonyle et cycloalcoyle; des groupes hétérocycliques saturés ou non contenant un atome d'oxygène, de soufre ou d'azote, et ayant 5 ou 6 atomes dans le cycle; des groupes de formule $-O-(CH_2CH_2O)_n$ alcoyle où n va de 1 à 4; et des groupes phényle éventuellement substitués; les substituants éventuels dans une portion cycloalcoyle éventuellement substituée étant choisis parmi des groupes alcoyle et haloalcoyle et les substituants énumérés à propos d'une portion alcoyle; et les substituants éventuels dans un groupe phényle éventuellement substitué étant choisis parmi des atomes d'halogènes et des groupes alcoyle, haloalcoyle, alcoxy, haloalcoxy, alcényle, haloalcényle, nitro et cyano; une portion alcoyle, alcényle ou alcynyle ayant jusqu'à 6 atomes de carbone; un groupe cycloalcoyle ayant 3 à 7 atomes de carbone dans le cycle; à ceci près que quand R représente un groupe alcoyle éventuellement substitué, la portion alcoyle peut contenir 1 à 20 atomes de carbone, et quand $R^1$ représente un groupe alcoyle ou haloalcoyle, l'halogène étant du chlore ou du fluor, le groupe alcoyle ou haloalcoyle peut contenir 1 à 10 atomes de carbone.

2. Un composé selon la revendication 1, dans lequel P et Q représentent chacun indépendamment un groupe alcoyle en $C_{1-4}$ ou un atome de fluor, de chlore ou de brome, et m est 0 ou 1.

3. Un composé selon la revendication 1 ou la revendication 2, dans lequel T représente un atome de brome, de fluor ou de chlore; un groupe $C_{1-4}$ alcoyle, haloalcoyle ou haloalcoxy; un groupe phénoxy éventuellement substitué dans lequel les substituants éventuels sont choisis parmi des groupes $C_{1-4}$ alcoyle et haloalcoyle, des atomes de fluor et de chlore et des groupes nitro et cyano; ou un groupe pyridyloxy éventuellement substitué dans lequel les substituants éventuels sont choisis parmi des groupes trifluorométhyle et des atomes de fluor et de chlore.

4. Un composé selon l'une quelconque des revendications 1 à 3, dans lequel T représente un groupe 2-pyridyloxy éventuellement substitué dans lequel les substituants éventuels sont choisis parmi des atomes d'halogènes et des groupes haloalcoyle, ou T représente un groupe 2-chloro-4-trifluorométhylphénoxy, 2-fluoro-4-trifluorométhylphénoxy ou 2-cyano-4-trifluorométhylphénoxy.

5. Un composé selon l'une quelconque des revendications 1 à 4, dans lequel Y représente un atome de fluor, de chlore ou de brome, un groupe $C_{1-4}$ alcoyle ou haloalcoyle ou un groupe nitro ou cyano et p est 0, 1 ou 2.

6. Un composé selon l'une quelconque des revendications 1 à 5, dans lequel X représente un atome de brome, de chlore ou de fluor ou un groupe alcoyle en $C_{1-4}$ et q est 0 ou 1.

7. Un composé selon l'une quelconque des revendications 1 à 6, dans lequel R représente un groupe alcoyle éventuellement substitué contenant 1 à 20 atomes de carbone un groupe cycloalcoyle en $C_{3-6}$ ou un groupe phényle éventuellement substitué, les substituants dans le groupe phényle éventuellement substitué étant choisis parmi des groupes $C_{1-4}$ alcoyle et haloalcoyle, des groupes nitro et cyano et des atomes d'halogènes, et les substituants dans un groupe alcoyle éventuellement substitué étant choisis parmi des atomes d'halogènes, des groupes alcoxy en $C_{1-4}$, des groupes carboxy, des groupes alcoxy-carbonyl contenant 2 à 5 atomes de carbone, des groupes de formule $-O-(CH_2CH_2O)_n$alcoyle où n va de 1 à 4, et la portion alcoyle a 1 à 4 atomes de carbone, des groupes phényle éventuellement substitués dans lesquels les substituants éventuels sont tels qu'indiqué ci-dessus pour un groupe phényle éventuellement substitué R, et des groupes hétérocycliques saturés ou non contenant un atome d'oxygène, de soufre ou d'azote et ayant 5 ou 6 atomes dans le cycle.

8. Un composé selon la revendication 7, dans lequel R représente un groupe alcoyle non substitué ayant de 1 à 10 atomes de carbone.

9. Un composé selon l'une quelconque des revendications 1 à 6, dans lequel $R^1$ représente un groupe alcoyle ou haloalcoyle contenant 1 à 10 atomes de carbone, l'halogène étant du chlore ou du fluor, et n est 1 ou 2.

10. Un composé selon l'une quelconque des revendications 1 à 9, dans lequel le groupe —COOR est situé dans la position 2 du groupe phényle auquel il est attaché.

11. Un procédé de préparation d'un composé tel que revendiqué dans l'une quelconque des revendications 1 à 10, qui comprend la réaction d'un benzoylisocyanate de formule:

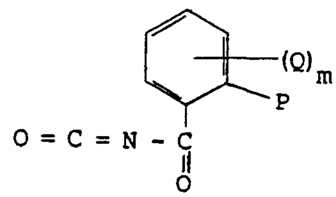

(III)

avec un sulfénamide de formule:

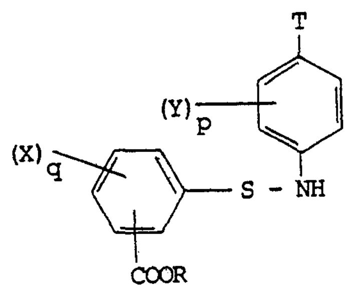

(IV)

où P, Q, m, R, X, q, T et Y ont les significations indiqueés dans l'une quelconque des revendications 1 à 10.

12. Un procédé selon la revendication 11, dans lequel la température de réaction est comprise entre 0 et 100°C.

13. Une composition à activité pesticide qui comprend un composé selon l'une quelconque des revendications 1 à 10, en même temps qu'un véhicule.

14. Une composition selon la revendication 13, qui comprend au moins deux véhicules, dont l'un au moins est un agent tensio-actif.

15. Un procédé de lutte contre des organismes nuisibles en un lieu, qui comprend l'application en ce lieu d'une quantité efficace du point de vue insecticide d'un composé selon l'une quelconque des revendications 1 à 10 ou d'une composition selon la revendication 13 ou la revendication 14.

**Revendications pour l'Etat Contactant: AT**

1. Un procédé pour la préparation d'un dérivé de benzoylurée de formule:

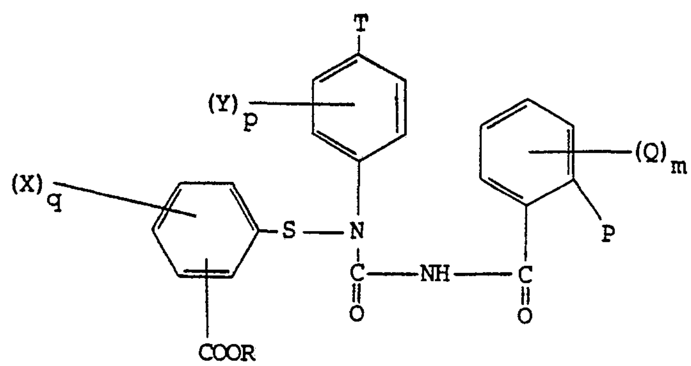

(I)

où P et Q représentent chacun indépendamment un atome d'halogène ou un groupe alcoyle; m représente 0, 1 ou 2; R représente un groupe alcoyle, alcényle, alcynyle, cycloalcoyle ou phényle éventuellement substitué, un atome d'hydrogène, un équivalent d'un métal alcalin ou d'un métal alcalino-terreux, un groupe ammonium ou ammonium substitué ou un groupe de formule générale $-(CH_2CH_2O)_nR^1$ où $R^1$ représente un groupe alcoyle, alcényle, alcynyle, cycloalcoyle ou phényle éventuellement substitué et n représente 1, 2, 3, 4 ou 5; chaque X indépendamment représente un atome d'halogène, un groupe cyano, nitro ou carboxy ou un groupe alcoyle, alcoxy, alcoylthio, cycloalcoyle, cycloalcoxy, cycloalcoylthio, alcényle, alcénylthio, alcoylcarbonyle, alcoxycarbonyle, alcoylsulfonyle, alcynyle, phényle, phénoxy, phénylthio ou amino; q = 0, 1, 2, 3 ou 4; T représente un atome d'halogène, un groupe alcoyle, haloalcoyle, alcoxy, haloalcoxy, alcényloxy ou haloalcényloxy ou un groupe phénoxy ou pyridyloxy éventuellement substitué par un ou plusieurs substituants choisi parmi des atomes d'halogènes et des groupes nitro, alcoyle, haloalcoyle et cyano; chaque Y indépendamment représente un atome d'halogène ou un groupe nitro, cyano, alcoyle ou haloalcoyle; et p = 0, 1, 2, 3 ou 4; les substituants éventuels dans une portion alcoyle, alcényle ou alcynyle éventuellement substituée étant choisis parmi des atomes d'halogènes; des groupes alcoxy, haloalcoxy, hydroxy, carboxy, alcoxycarbonyle et cycloalcoyle; des groupes hétérocycliques saturés ou non contenant un atome d'oxygène, de soufre ou d'azote et ayant 5 ou 6 atomes dans le cycle; des groupes de formule $-O-(CH_2CH_2O)_n$alcoyle où n va de 1 à 4; et des groupes phényle

éventuellement substitués; les substituants éventuels dans une portion cycloalcoyle éventuellement substituée étant choisis parmi des groupes alcoyle et haloalcoyle et les groupes substituants indiqués à propos des groupes alcoyle; et les substituants éventuels dans un groupe phényle éventuellement substitué étant choisis parmi des atomes d'halogènes et des groupes alcoyle, haloalcoyle, alcoxy, haloalcoxy, alcényle, haloalcényle, nitro et cyano; une portion alcoyle, alcényle ou alcynyle ayant jusqu'à 6 atomes de carbone; un groupe cycloalcoyle ayant 3 à 7 atomes de carbone dans le cycle; à ceci près que quand R représente un groupe alcoyle éventuellement substitué, la portion alcoyle peut contenir 1 à 20 atomes de carbone, et quand $R^1$ représente un groupe alcoyle ou haloalcoyle, l'halogène étant du chlore ou du fluor, le groupe alcoyle ou haloalcoyle peut contenir 1 à 10 atomes de carbone, qui comprend la réaction d'un benzoylisocyanate de formule:

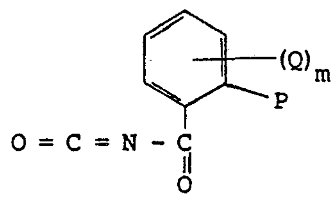

(III)

avec un sulfénamide de formule:

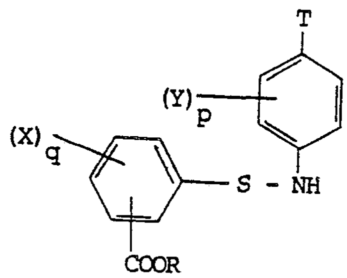

(IV)

où P, Q, m, R, X, q, T, Y et p ont les significations indiquées ci-dessus.

2. Un procédé selon la revendication 1, dans lequel P et Q représentent chacun indépendamment un groupe alcoyle en $C_{1-4}$ ou un atome de fluor, de chlore ou de brome, et m est 0 ou 1.

3. Un procédé selon la revendication 1 ou la revendication 2, dans lequel T représente un atome de brome, de fluor ou de chlore; un groupe $C_{1-4}$ alcoyle, haloalcoyle ou haloalcoxy; un groupe phénoxy éventuellement substitué dans lequel les substituants éventuels sont choisis parmi des groupes $C_{1-4}$ alcoyle et haloalcoyle, des atomes de fluor et de chlore et des groupes nitro et cyano; ou un groupe pyridyloxy éventuellement substitué dans lequel les substituants éventuels sont choisis parmi des groupes trifluorométhyle et des atomes de fluor et de chlore.

4. Un procédé selon l'une quelconque des revendications 1 à 3, dans lequel T représente un groupe 2-pyridyloxy éventuellement substitué dans lequel les substituants éventuels sont choisis parmi des atomes d'halogènes et des groupes haloalcoyle, ou T représente un groupe 2-chloro-4-trifluorométhylphénoxy, 2-fluoro-4-trifluorométhylphénoxy ou 2-cyano-4-trifluorométhylphénoxy.

5. Un procédé selon l'une quelconque des revendications 1 à 4, dans lequel Y représente un atome de fluor, de chlore ou de brome, un groupe $C_{1-4}$ alcoyle ou haloalcoyle ou un groupe nitro ou cyano et p est 0, 1 ou 2.

6. Un procédé selon l'une quelconque des revendications 1 à 5, dans lequel X représente un atome de brome, de chlore ou de fluor ou un groupe alcoyle en $C_{1-4}$ et q est 0 ou 1.

7. Un procédé selon l'une quelconque des revendications 1 à 6, dans lequel R représente un groupe alcoyle éventuellement substitué contenant 1 à 20 atomes de carbone un groupe cycloalcoyle en $C_{3-6}$ ou un groupe phényle éventuellement substitué, les substituants dans le groupe phényle éventuellement substitué étant choisis parmi des groupes $C_{1-4}$ alcoyle et haloalcoyle, des groupes nitro et cyano et des atomes d'halogènes et les substituants dans un groupe alcoyle éventuellement substitué étant choisis parmi des atomes d'halogènes, des groupes alcoxy en $C_{1-4}$, des groupes carboxy, des groupes alcoxy-carbonyl contenant 2 à 5 atomes de carbone, des groupes de formule $-O-(CH_2CH_2O)_n$alcoyle où n va de 1 à 4, et la portion alcoyle a 1 à 4 atomes de carbone, des groupes phényle éventuellement substitués dans lesquels les substituants éventuels sont tels qu'indiqué ci-dessus pour un groupe phényle éventuellement substitué R, et des groupes hétérocycliques saturés ou non contenant un atome d'oxygène, de soufre ou d'azote et ayant 5 ou 6 atomes dans le cycle.

8. Un procédé selon la revendication 7, dans lequel R représente un groupe alcoyle non substitué ayant de 1 à 10 atomes de carbone.

9. Un procédé selon l'une quelconque des revendications 1 à 6, dans lequel $R^1$ représente un groupe alcoyle ou haloalcoyle contenant 1 à 10 atomes de carbone, l'halogène étant du chlore ou du fluor, et n est 1 ou 2.

10. Un procédé selon l'une quelconque des revendications 1 à 9, dans lequel le groupe —COOR est situé dans la position 2 du groupe phényle auquel il est attaché.

11. Un procédé selon l'une quelconque des revendications 1 à 10, dans lequel la température de réaction est comprise entre 0 et 100°C.

12. Une composition à activité pesticide qui comprend un composé selon de formule I tel que défini dans l'une quelconque des revendications 1 à 10, en même temps qu'un véhicule.

13. Une composition selon la revendication 12, qui comprend au moins deux véhicules, dont l'un au moins est un agent tensio-actif.

14. Un procédé de lutte contre des organismes nuisibles en un lieu, qui comprend l'application en ce lieu d'une quantité efficace du point de vue insecticide d'un composé de formule I tel que défini dans l'une quelconque des revendications 1 à 10 ou d'une composition selon la revendication 12 ou la revendication 13.